(19) Europäisches Patentamt
European Patent Office
Office européen des brevets

(11) EP 0 748 326 B1

(12) **FASCICULE DE BREVET EUROPEEN**

(45) Date de publication et mention de la délivrance du brevet:
**04.07.2001 Bulletin 2001/27**

(21) Numéro de dépôt: **95943260.0**

(22) Date de dépôt: **22.12.1995**

(51) Int Cl.[7]: **C07F 9/6568**, C07F 15/00, C07C 51/36, C07B 53/00, C07C 57/00 // C07M7:00

(86) Numéro de dépôt international:
**PCT/FR95/01716**

(87) Numéro de publication internationale:
**WO 96/20202 (04.07.1996 Gazette 1996/30)**

(54) **DIPHOSPHINES OPTIQUEMENT ACTIVES, LEUR PREPARATION PAR DEDOUBLEMENT DU MELANGE RACEMIQUE**

OPTISCH AKTIVE DIPHOSPHINE UND HERSTELLUNGSVERFAHREN DURCH AUFTRENNUNG DER RACEMATMISCHUNG

OPTICALLY ACTIVE DIPHOSPHINES AND METHOD FOR PREPARING SAME BY RESOLUTION OF THE RACEMIC MIXTURE

(84) Etats contractants désignés:
**AT BE CH DE DK ES FR GB IE IT LI NL PT SE**

(30) Priorité: **28.12.1994 FR 9415757**
**29.05.1995 FR 9506286**

(43) Date de publication de la demande:
**18.12.1996 Bulletin 1996/51**

(73) Titulaire: **RHODIA CHIMIE**
**92408 Courbevoie Cédex (FR)**

(72) Inventeurs:
- **MATHEY, François**
  **F-75004 Paris (FR)**
- **ROBIN, Frédéric**
  **F-92120 Montrouge (FR)**
- **MERCIER, François**
  **F-78000 Versailles (FR)**
- **SPAGNOL, Michel**
  **F-69003 Lyon (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
- **BULL. SOC. CHIM. FR. (BSCFAS,00378968);92; VOL.129 (1); PP.1-8, EC. POLYTECH.;LAB. CHIM. PHOSPHORE MET. TRANSITION; PALAISEAU; 91128; FR. (FR), BEVIERRE M O ET AL 'Mechanism of the thermal tetramerization of phospholes' cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a trait à des diphosphines optiquement actives. Elle vise également leur préparation selon un procédé de dédoublement du mélange racémique desdites phosphines en isomères optiquement actifs.

Plus précisément, l'invention a pour objet de nouvelles diphosphines optiquement actives de bis-[1-phospha-2,3-diphényl-4,5-diméthylnorbornadiène] et le procédé de dédoublement du mélange racémique de bis-[1-phospha-2,3-diphényl-4,5-diméthylnorbornadiène].

L'invention concerne également de nouveaux complexes métalliques optiquement actifs comprenant lesdites phosphines et leur mise en oeuvre dans un procédé de préparation d'acides carboxyliques et/ou dérivés, optiquement actifs, selon un procédé d'hydrogénation d'acides carboxyliques $\alpha,\beta$ insaturés et/ou dérivés.

Il a été décrit par F. Mathey et al, dans Bull. Soc. Chim. Fr. 129, pp.1-8 (1992) la préparation d'un mélange de diastéréoisoméres de bis-[1-phospha-2,3-diphényl-4,5-diméthylnorbornadiène].

Le produit de départ de la synthèse de ceux-ci est le 1-phényl-3,4-diméthylphosphole (II) décrit par F. Mathey et al, dans Synthesis, 1983, pp. 983.

On commence par réaliser la préparation du 3,3',4,4'-tétraméthyl-1,1'-diphosphole (IV). A cet effet, on fait réagir le 1-phényl-3,4-diméthylphosphole (II) dans le THF, avec du lithium métal suivant la réaction suivante :

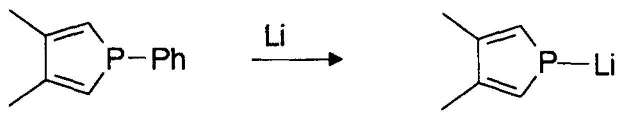


(II) (III)

On introduit en fin de réaction du chlorure d'aluminium pour piéger le phényllithium produit au cours de la réaction.

Dans une étape suivante, on réalise la dimérisation de (III) par action du diiode $I_2$ dans le THF. Pour plus de détails sur la préparation de (IV), on peut se reporter à l'article de F. Mathey et al, Organometallics, 1983, 2, 1234.

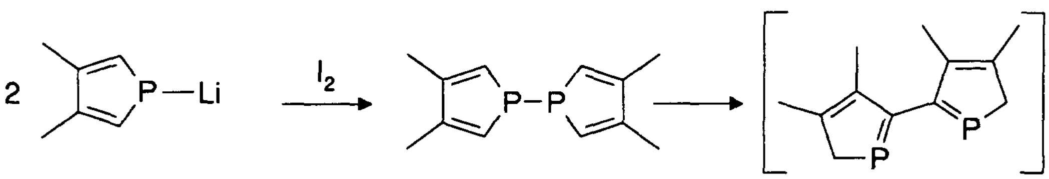


(III) (IV) (V)

Par chauffage vers 140°C, le composé (IV) se réarrange en (V) qui réagit avec le diphénylacétylène selon Diels-Alder, pour donner le bis-[1-phospha-2,3-diphényl-4,5-diméthylnorbornadiène].

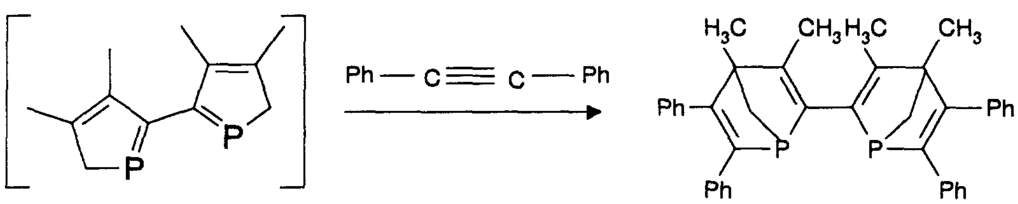

(V) (I)

Un mode de réalisation pratique est donné page 6 de la publication F. Mathey et al, dans Bull. Soc. Chim. Fr. 129, pp.1-8 (1992).

Toutefois, les auteurs ont obtenu comme mentionné page 3 colonne droite, lignes 7 et 8, un mélange de deux diastéréoisomères identifiés postérieurement par la demanderesse comme étant un méso (I m) - RS, SR - et un racémique (I r) - RR, SS - appelés respectivement dans l'article, (13b) et (13a).

La publication mentionne la séparation des deux diastéréoisomères par formation d'un chélate de palladium (II). Pour ce faire, il est décrit la séparation du mélange de diastéréoisomères obtenus, par réaction avec $PdCl_2(PhCN)_2$

dans le dichloroéthane conduisant à (VI m) et (VI r), la séparation par chromatographie sur silicagel suivie d'une élution, puis d'une décomplexation effectuée par NaCN.

On récupère donc séparément les deux diastéréoisomères, d'une part le méso (I m) et d'autre part le racémique (I r).

Le document de l'état de la technique ne décrit pas la séparation des énantiomères.

Le problème du dédoublement de deux énantiomères est difficile à résoudre lorsque la chiralité est portée par le phosphore.

Un objectif de la présente invention est de fournir des nouvelles diphosphines optiquement actives, bidentées, chirales sur le phosphore et non racémisables.

Un autre objectif de la présente invention est de mettre à disposition un procédé permettant de les obtenir selon un procédé de dédoublement du mélange racémique de bis-[1-phospha-2,3-diphényl-4,5-diméthylnorbornadiène].

Enfin, un autre objectif de l'invention est de disposer d'un procédé de préparation d'acides carboxyliques et/ou dérivés, optiquement actifs, selon un procédé d'hydrogénation d'acides carboxyliques $\alpha,\beta$ insaturés et dérivés mettant en oeuvre des complexes métalliques utilisant comme ligand, la diphosphine optiquement active.

Selon un premier objet de la présente invention, il a été trouvé de nouvelles diphosphines optiquement actives de bis-[1-phospha-2,3-diphényl-4,5-diméthylnorbornadiène] répondant aux formules suivantes :

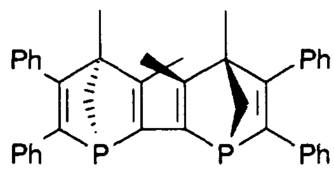

(Ia) - (S,S) - (+)

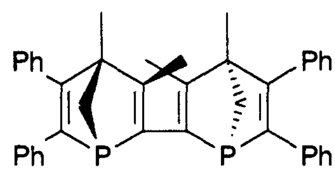

(Ib) - (R,R) - (-)

Selon un autre objet de l'invention, on effectue le dédoublement du mélange racémique de bis-[1-phospha-2,3-diphényl-4,5-diméthylnorbornadiène] selon un procédé qui consiste à le faire réagir avec un complexe de palladium et/ou de platine comme auxiliaire chiral, dans un solvant organique formant ainsi des complexes diastéréoisomères, puis à dédoubler lesdits complexes optiquement purs.

Conformément au procédé de l'invention, on fait appel à un complexe de palladium. Ce type d'auxiliaire chiral est largement décrit dans la littérature, notamment par Sei Otsuka et al, dans Journal of the American Chemical Society 93, pp. 4301 (1971).

On peut également faire appel à un complexe de platine et l'on peut se référer plus particulièrement aux travaux de A. C. Cope [Journal of the American Chemical Society 90, pp. 909 (1968)].

Le complexe chiral mis en oeuvre répond plus particulièrement à la formule générale (VII) :

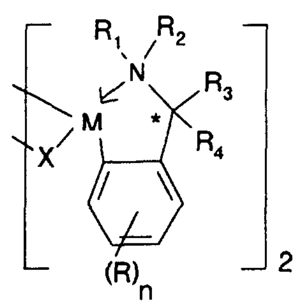

(VII)

dans ladite formule :

- M représente le palladium et/ou le platine,
- $R_1$, $R_2$, $R_3$ et $R_4$ représentent un atome d'hydrogène ou un radical alkyle ayant de 1 à 10 atomes de carbone ou un radical cycloalkyle ayant de 3 à 10 atomes de carbone,
- $R_3$ et $R_4$ sont différents et au moins l'un des deux représente un atome d'hydrogène,
- R a la signification donnée pour $R_1$, $R_2$, $R_3$ et $R_4$,
- X représente un atome d'halogène,
- n est un nombre de 0 à 4,
- lorsque n est supérieur à 1, deux radicaux R et les 2 atomes successifs du cycle benzénique peuvent former entre eux, un cycle ayant de 5 à 7 atomes de carbone.

Plus préférentiellement, le complexe mis en oeuvre répond à la formule précitée dans laquelle $R_1$, $R_2$, $R_3$ et $R_4$ représentent un atome d'hydrogène ou un radical méthyle, X représente un atome de chlore et n est égal à 0.
Lorsque n est égal à 2, deux radicaux R forment un cycle benzénique.
Comme exemples plus spécifiques de complexes de palladium convenant à la présente invention obtenus indifféremment à partir de (R)-(+) ou (S)-(-)-N,N-diméthylphényléthylamine, on peut mentionner :

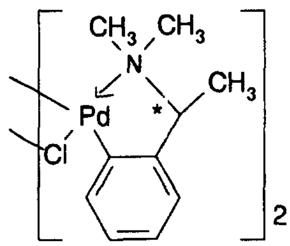

(VII')

La quantité de complexe des métaux précités exprimée en métal est généralement de 0,5 à 1 atome de métal par atome de phosphore.
On fait appel à un solvant organique qui solubilise tous les réactifs. Le solvant doit être inerte vis-à-vis de la diphosphine.
A titre d'exemples non limitatifs de solvants convenant dans le procédé de l'invention, on peut citer :

- les hydrocarbures aliphatiques et plus particulièrement les paraffines tels que notamment, le pentane, l'hexane, l'heptane, l'octane, l'isooctane, le nonane, le décane, l'undécane, le tétradécane, l'éther de pétrole et le cyclohexane ; les hydrocarbures aromatiques comme notamment le benzène, le toluène, les xylènes, l'éthylbenzène, les diéthylbenzènes, les triméthylbenzènes, le cumène, le pseudocumène, les coupes pétrolières constituées d'un mélange d'alkylbenzènes notamment les coupes de type Solvesso® .
- les hydrocarbures halogénés aliphatiques ou aromatiques, et l'on peut mentionner : les hydrocarbures perchlorés tels que notamment le trichlorométhane, le tétrachloroéthylène ; les hydrocarbures partiellement chlorés tels que le dichlorométhane, le dichloroéthane, le tétrachloroéthane, le trichloroéthylène, le 1-chlorobutane, le 1,2-dichlorobutane ; le monochlorobenzène, le 1,2-dichlorobenzène, le 1,3-dichlorobenzène, le 1,4-dichlorobenzène ou des mélanges de différents chlorobenzènes.

Parmi tous ces solvants, le benzène et le toluène sont préférés.
La concentration de la diphosphine dans le solvant réactionnel est de préférence, entre 0,05 et 1 mole/litre et encore plus particulièrement entre 0,05 et 0,2 mole/litre.
La séparation est avantageusement conduite à température ambiante généralement comprise entre 15°C et 25°C.
Elle a lieu de préférence sous atmosphère contrôlée de gaz inertes. On peut établir une atmosphère de gaz rares, de préférence l'argon mais il est plus économique de faire appel à l'azote.
On obtient un mélange de complexes de palladium ou platine et de diphosphine correspondant à chaque énantiomère.
Un autre objet de l'invention est le produit intermédiaire à savoir le complexe métallique avec les diphosphines de formule :

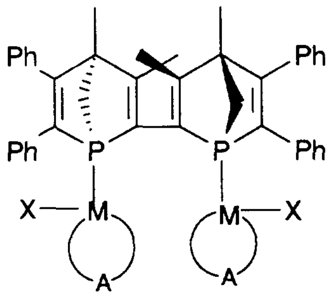 (VIII a) 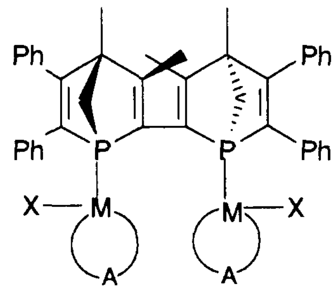 (VIII b)

dans lesdites formules, M représente le palladium ou le platine, X un atome d'halogène, de préférence, le chlore et A symbolise le reste d'un complexe métallique chiral répondant à l'une des formules (VII) et préférentiellement (VII').

Dans une étape suivante, on récupère les deux énantiomères purs.

On concentre par évaporation le solvant puis on effectue la séparation d'une manière connue [A. Bertheillier - Dunod Paris (1972)] par chromatographie liquide sur colonne, avec, de préférence un support en silice.

On élue la colonne avec un mélange de solvants appropriés, de préférence, un mélange toluène/acétate d'éthyle comprenant préférentiellement, 80 % en volume de toluène et 20 % en volume d'acétate d'éthyle.

On récupère les deux énantiomères isolés, purs, sous forme de deux complexes diastéréoisomères ayant les caractéristiques suivantes :

RMN$^{31}$P = $\delta(CH_2Cl_2)$ = 55,9 ppm
RMN$^{31}$P = $\delta(CH_2Cl_2)$ = 53,6 ppm

On récupère les deux énantiomères de la diphosphine purs en effectuant la décomplexation.

A cet effet, on utilise notamment un sel de l'acide cyanhydrique, de préférence, un sel alcalin et encore plus préférentiellement le sodium : ledit sel étant mis en solution dans le minimum d'eau nécessaire.

On solubilise les complexes dans un solvant organique tel que, par exemple, le dichlorométhane, puis l'on introduit sous agitation, le sel de l'acide cyanhydrique mis en oeuvre généralement en excès représentant de 2 à 5 moles par atome de métal.

L'opération est également conduite sous atmosphère contrôlée et à température ambiante.

On récupère l'énantiomère dans la phase organique qui est séparée, lavée à l'eau et séchée, par exemple sur sulfate de sodium.

On obtient les deux énantiomères du bis-[1-phospha-2,3-diphényl-4,5-diméthylnorbornadiène], isolés purs répondant aux formules [(Ia) - (S,S) (+)] et [(Ib) - (R,R) (-)] précitées, dont les caractéristiques sont les suivantes :

RMN$^{31}$P = $\delta(CDCl_3)$ = - 13,2 ppm - $[\alpha]_D$ = + 231° (c = 1, $C_6D_6$).
RMN$^{31}$P = $\delta(CDCl_3)$= - 13,2 ppm - $[\alpha]_D$ = - 198° (c = 1, $C_6D_6$)
(avec un $[\alpha]_D$ déterminé pour une concentration de 10 mg/ml et à température ambiante).

Il a également été trouvé et c'est ce qui constitue un autre objet de la présente invention, que les nouvelles diphosphines optiquement actives telles que précitées à l'état de complexes métalliques, pouvaient être utilisées comme catalyseurs d'hydrogénation asymétrique d'acides carboxyliques $\alpha,\beta$ insaturés et/ou dérivés.

Les diphosphines optiquement actives de formule (Ia) ou (Ib) servent de ligands dans la formation de complexes avec des métaux de transition.

Un objet de l'invention est donc de nouveaux complexes comprenant une diphosphine optiquement active et un métal de transition qui sont caractérisés par le fait que le ligand répond à l'une des formules suivantes :

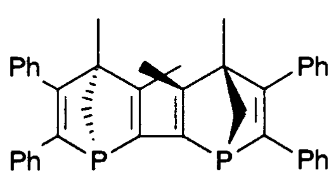 (Ia) - (S,S) - (+)

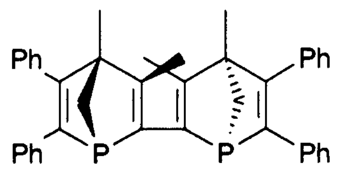
(Ib) - (R,R) - (-)

Comme exemples de métaux de transition capables de former des complexes, on peut citer notamment les métaux tels le rhodium, le ruthénium, le rhénium, l'iridium, le cobalt, le nickel, le platine, le palladium.
Parmi les métaux précités, le rhodium, le ruthénium et l'iridium sont préférés.
Des exemples spécifiques desdits complexes de la présente invention sont donnés ci-après, sans caractère limitatif.
Dans lesdites formules, (P*P) représente la diphosphine de formule (Ia) ou (Ib).
Les complexes de rhodium et d'iridium peuvent être représentés par les formules suivantes :

$$[M\ L_2(P^*P)]\ Y \qquad (IIa)$$

$$[M\ L_2(P^*P)]\ Y \qquad (IIb)$$

dans lesdites formules :

- (P*P) représente dans la formule (IIa) la diphosphine de formule (Ia) et dans la formule (IIb) la diphosphine de formule (Ib),
- M représente le rhodium ou l'iridium,
- Y représente un ligand anionique coordinant,
- L représente un ligand neutre.

Les complexes préférés de rhodium ou d'iridium répondent à la formule (IIa) ou (IIb) dans laquelle :

- L représente une oléfine ayant de 2 à 12 atomes de carbone et deux ligands L peuvent être liés entre eux pour former une chaîne hydrocarbonée polyinsaturée, linéaire ou cyclique ; L représentant de préférence, le 1,5-cyclooctadiène, le norbornadiène, l'éthylène,
- Y représente un anion $PF_6^-$, $PCl_6^-$, $BF_4^-$, $BCl_4^-$, $SbF_6^-$, $SbCl_6^-$, $BPh_4^-$, $ClO_4^-$, CN-, $CF_3SO_3^-$, halogène de préférence, $Cl^-$ ou $Br^-$, un anion 1,3-dicétonate, alkylcarboxylate, haloalkylcarboxylate avec un radical alkyle inférieure, un anion phénylcarboxylate ou phénolate dont le cycle benzénique peut être substitué par des radicaux alkyle inférieurs et/ou des atomes d'halogène.

Par radicaux alkyle inférieurs, on entend généralement un radical alkyle linéaire ou ramifié ayant de 1 à 4 atomes de carbone.
D'autres complexes d'iridium peuvent être représentés par les formules :

$$[Ir\ L\ (P^*P)]\ Y \qquad (IIIa)$$

$$[Ir\ L\ (P^*P)]\ Y \qquad (IIIb)$$

dans lesdites formules, (P*P), L et Y ont les significations données pour les formules (IIa) et (IIb).
Pour ce qui est des complexes de ruthénium, ils répondent préférentiellement aux formules suivantes :

$$[RuY_1Y_2(P^*P)] \qquad (IVa)$$

$$[RuY_1Y_2(P^*P)] \quad (IVb)$$

dans lesdites formules :

- (P*P) représente dans la formule (IVa) la diphosphine de formule (Ia) et dans la formule (IVb) la diphosphine de formule (Ib),
- $Y_1$ et $Y_2$, identiques ou différents, représentent de préférence, un anion $PF_6$-, $PCl_6^-$, $BF_4^-$, $BCl_4^-$, $SbF_6^-$, $SbCl_6^-$, $BPh_4^-$, $ClO_4^-$, $CF_3SO_3^-$, un atome d'halogène, plus particulièrement, chlore ou brome ou un anion carboxylate, préférentiellement, acétate, trifluoroacétate.

D'autres complexes du ruthénium susceptibles d'être mis en oeuvre dans la procédé de l'invention répondent aux formules ci-après :

$$[RuY_1Ar(P^*P)Y_2] \quad (IVc)$$

$$[RuY_1Ar(P^*P)Y_2] \quad (IVd)$$

dans lesdites formules :

- (P*P) représente dans la formule (IVc) la diphosphine de formule (Ia) et dans la formule (IVd) la diphosphine de formule (Ib),
- Ar représente le benzène, le p-méthylisopropylbenzène, l'hexaméthylbenzène,
- $Y_1$ représente un atome d'halogène, de préférence, chlore ou brome,
- $Y_2$ représente un anion de préférence, un anion $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$.

Il est également possible de mettre en oeuvre dans le procédé de l'invention des complexes à base de palladium et de platine.

Comme exemples plus spécifiques desdits complexes, on peut mentionner entre autres $PdCl_2(P^*P)$ et $PtCl_2$ $(P^*P)$ dans lesquels (P*P) représente la diphosphine de formule (Ia) ou (Ib).

Les complexes comprenant la diphosphine précitée et le métal de transition peuvent être préparés selon les procédés connus décrits dans la littérature.

Pour la préparation des complexes de ruthénium, on peut se référer notamment à la publication de J.-P. Genêt [Acros Organics Acta, 1, Nr. 1, pp. 1-8 (1994)] et pour les autres complexes, à l'article de Schrock R. et Osborn J.A. [Journal of the American Chemical Society, 93, pp. 2397 (1971)].

Ils peuvent être préparés en particulier par réaction de la diphosphine de formule (Ia) ou (Ib) avec le composé de métal de transition, dans un solvant organique approprié.

La réaction est conduite à une température comprise entre la température ambiante (de 15 à 25°C) et la température de reflux du solvant réactionnel.

Comme exemples de solvants organiques, on peut mentionner entre autres, les hydrocarbures aliphatiques, halogénés ou non et plus particulièrement, l'hexane, l'heptane, l'isooctane, le décane, le benzène, le toluène, le chlorure de méthylène, le chloroforme ; des solvants de type éther ou cétone et notamment le diéthyléther, le tétrahydrofurane, l'acétone, la méthyléthylcétone ; les solvants de type alcool, de préférence, le méthanol ou l'éthanol.

Les complexes métalliques selon l'invention, récupérés selon les techniques classiques (filtration ou cristallisation) sont utilisés dans des réactions d'hydrogénation asymétrique de substrats précisés ci-après.

Un autre objet de la présente invention est de fournir un procédé de préparation d'un acide carboxylique et/ou dérivé, optiquement actif, procédé qui est caractérisé par le fait que l'on effectue l'hydrogénation asymétrique d'un acide carboxylique $\alpha,\beta$-insaturé et/ou de ses dérivés en présence d'une quantité efficace d'un complexe métallique comprenant à titre de ligand, la diphosphine optiquement active de formule (Ia) ou (Ib) et un métal de transition.

L'acide carboxylique $\alpha,\beta$-insaturé et/ou ses dérivés répond plus particulièrement à la formule (V) :

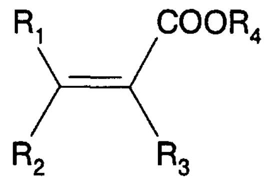

(V)

dans ladite formule (V) :

- $R_1$, $R_2$, $R_3$ et $R_4$, représentent un atome d'hydrogène ou n'importe quel groupe hydrocarboné, dans la mesure où :
  . si $R_1$ est différent de $R_2$ et différent d'un atome d'hydrogène alors $R_3$ peut être n'importe quel groupe hydrocarboné ou fonctionnel désigné par $\mathcal{R}$ ,
  . si $R_1$ ou $R_2$ représente un atome d'hydrogène et si $R_1$ est différent de $R_2$, alors $R_3$ est différent d'un atome d'hydrogène et différent de $-COOR_4$,
  . si $R_1$ est identique à $R_2$ et représente n'importe quel groupe hydrocarboné ou fonctionnel désigné par $\mathcal{R}$ , alors $R_3$ est différent de $-CH-(\mathcal{R})_2$ et différent de $-COOR_4$,
- l'un des groupes $R_1$, $R_2$ et $R_3$ pouvant représenter un groupe fonctionnel.

Les radicaux $R_1$ à $R_4$ identiques ou différents représentent un radical hydrocarboné éventuellement substitué ayant de 1 à 20 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique ; un radical aliphatique saturé ou insaturé, linéaire ou ramifié, porteur d'un substituant cyclique.

Dans la formule générale (V), $R_1$ à $R_4$, identiques ou différents peuvent prendre diverses significations. Différents exemples sont donnés ci-après mais ils ne sont en aucun cas limitatif.

Ainsi, les radicaux $R_1$ à $R_4$ représentent préférentiellement un radical hydrocarboné aromatique, et notamment benzénique répondant à la formule générale (V') :

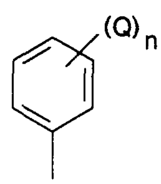

(V')

dans ladite formule (V') :

- n est un nombre entier de 0 à 5, de préférence de 0 à 3,
- Q représente $R_0$, l'un des groupes ou fonctions suivantes :
  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_5-OH$$

$$-R_5-COOR_7$$

$-R_5-CHO$

$-R_5-NO_2$

$-R_5-CN$

$-R_5-N(R_7)_2$

$-R_5-CO-N(R_7)_2$

$-R_5-SH$

$-R_5-X$

$-R_5-CF_3$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_7$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- Q représente $R_0$', l'un des radicaux plus complexes suivants :

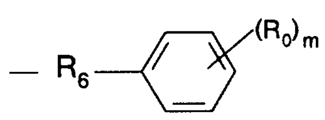

dans lequel :

- m est un nombre entier de 0 à 5, de préférence de 0 à 3,
  - . $R_0$ a la signification donnée précédemment,
  - . $R_6$ représente un lien valentiel ; un groupe hydrocarboné divalent, linéaire ou ramifié, saturé ou insaturé ayant de 1 à 6 atomes de carbone tel que par exemple, méthylène, éthylène, propylène, isopropylène, isopropylidène ou l'un des groupes suivants dénommés Z:
    $-O-$ ; $-CO-$ ; $-COO-$ ; $-NR_7-$ ; $-CO-NR_7-$; $-S-$ ; $-SO_2-$ ; $-NR_7-CO-$;
    dans lesdites formules $R_7$ représente un atome d'hydrogène, un groupe alkyle linéaire ou ramifié ayant de 1 à 6 atomes de carbone, de préférence, un radical méthyle ou éthyle.

Lorsque n est supérieur à 1, les radicaux Q peuvent être identiques ou différents et 2 atomes de carbone successifs du cycle benzénique peuvent être reliés entre eux par un pont cétalique tels que les radicaux méthylène dioxy ou éthylène dioxy extranucléaires.

De préférence, n est égal à 0, 1, 2 ou 3.

Parmi tous les radicaux $R_1$ à $R_4$ précités, on met en oeuvre tout préférentiellement dans le procédé de l'invention, les acides carboxyliques ou dérivés répondant à la formule générale (V) dans laquelle $R_1$ à $R_4$ représentent un radical aromatique répondant à la formule générale (V') dans laquelle :

- n est égal à 0, 1, 2 ou 3,
- Q représente l'un des groupes ou fonctions suivantes :

  . un atome d'hydrogène,
  . un radical alkyle linéaire ou ramifié, ayant de 1 à 4 atomes de carbone,
  . un radical alkoxy linéaire ou ramifié ayant de 1 à 4 atomes de carbone,
  . un groupe benzoyle,
  . un groupe -OH,
  . un groupe -CHO,
  . un groupe $NH_2$,
  . un groupe $NO_2$,
  . un radical phényle,
  . un atome d'halogène,
  . un groupe $CF_3$.

Encore plus préférentiellement, on choisit les composés de formule (V) dans laquelle les radicaux Q identiques ou différents sont un atome d'hydrogène, un radical alkyle ayant de 1 à 4 atomes de carbone, un radical méthoxy, un groupe benzoyle, un groupe $NO_2$.

Comme exemples de radicaux $R_1$ à $R_4$ répondant à la formule (V), on peut mentionner plus précisément les radicaux phényle, tolyle ou xylyle, 1-méthoxyphényle, 2-nitrophényle et les radicaux biphényle, 1,1'-méthylènebiphényle, 1,1'-isopropylidènebiphényle, 1,1'-carboxybiphényle, 1,1'-oxybiphényle, 1,1'-iminobiphényle: lesdits radicaux pouvant être substitués par un ou plusieurs radicaux Q tels que prédéfini.

$R_1$ à $R_4$ peuvent également représenter un radical hydrocarboné aromatique polycyclique ; les cycles pouvant former entre eux des systèmes ortho-condensés, ortho- et péri-condensés. On peut citer plus particulièrement, un radical naphtalénique ; lesdits cycles pouvant être substitués par 1 à 4 radicaux $R_0$, de préférence 1 à 3, $R_0$ ayant les significations énoncées précédemment pour les substituants du radical hydrocarboné aromatique de formule générale (V').

Dans la formule générale (V) des acides carboxyliques, $R_1$ à $R_4$ peuvent représenter également un radical carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux $R_0$, de préférence 1 à 3, $R_0$ ayant les significations énoncées précédemment pour les substituants du radical hydrocarboné aromatique de formule générale (V').

Comme exemples préférés de radicaux $R_1$ à $R_4$, on peut citer les radicaux cyclohexyle ou cyclohexène-yle, éventuellement substitué par des radicaux alkyle linéaires ou ramifiés, ayant de 1 à 4 atomes de carbone.

Comme mentionné précédemment, $R_1$ à $R_4$ peuvent représenter un radical aliphatique acyclique, saturé ou insaturé, linéaire ou ramifié.

Plus précisément, $R_1$ à $R_4$ représentent un radical aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

La chaîne hydrocarbonée peut être éventuellement :

- interrompue par l'un des groupes suivants Z :
  $-O-$ ; $-CO-$ ; $-COO-$ ; $-NR_7-$ ; $-CO-NR_7-$; $-S-$ ; $-SO_2-$ ; $-NR_7-CO-$;
  dans lesdites formules $R_7$ représente un atome d'hydrogène, un groupe alkyle linéaire ou ramifié ayant de 1 à 6 atomes de carbone, de préférence, un radical méthyle ou éthyle,
- et/ou porteuse de l'un des substituants suivants :
  $-OH$, $-COOR_7$, $-CHO$, $-NO_2$, $-CN$, $-NH_2$, $-SH$, $-X$, $-CF_3$,
  dans ces formules, $R_7$ ayant la signification donnée précédemment.

Il est également possible de faire appel à un acide carboxylique ou dérivé de formule (V) dans laquelle $R_1$ à $R_4$ représentent un radical aliphatique acyclique, saturé ou insaturé, linéaire ou ramifié pouvant être éventuellement porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes Z précités.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs de 1, 2, 3, 4 ou 5 radicaux $R_0$, identiques ou différents, $R_0$ ayant les significations énoncées précédemment pour les substituants du radical hydrocarboné aromatique de formule générale (V').

Comme exemples de tels radicaux, on peut mentionner, entre autres, le radical benzyle.

Dans la formule générale (V) des acides carboxyliques, $R_1$ à $R_4$ peuvent également représenter un radical hétérocyclique, saturé ou non, comportant notamment 5 ou 6 atomes dans le cycle dont 1 ou 2 hétéroatomes tels que les atomes d'azote, de soufre et d'oxygène ; les atomes de carbone de l'hétérocycle pouvant- éventuellement être substitués, dans leur totalité ou pour une partie d'entre eux seulement par des radicaux $R_0$, $R_0$ ayant les significations énoncées précédemment pour les substituants du radical hydrocarboné aromatique de formule générale (V').

$R_1$ à $R_4$ peuvent aussi représenter un radical hétérocyclique polycyclique défini comme étant soit un radical constitué par au moins 2 hétérocycles aromatiques ou non contenant au moins un hétéroatome dans chaque cycle et formant entre eux des systèmes ortho- ou ortho- et péri-condensés ou soit un radical constitué par au moins un cycle hydrocarboné aromatique ou non et au moins un hétérocycle aromatique ou non formant entre eux des systèmes ortho- ou ortho- et péri-condensés ; les atomes de carbone desdits cycles pouvant éventuellement être substitués, dans leur totalité ou pour une partie d'entre eux seulement par des radicaux $R_0$, $R_0$ ayant les significations énoncées précédemment pour les substituants du radical hydrocarboné aromatique de formule générale (V').

A titre d'exemples de groupements $R_1$ à $R_4$ de type hétérocyclique, on peut citer entre autres, les radicaux furyle, pyrrolyle, thiényle, isoxazolyle, furazannyle, isothiazolyle, imidazolyle, pyrazolyle, pyridyle, pyridazinyle, pyrimidinyle, pyrannyle et les radicaux quinolyle, naphtyridinyle, benzopyrannyle, benzofurannyle, indolyle.

Il est également possible que parmi les radicaux $R_1$ à $R_3$, l'un d'entre eux représente un groupement fonctionnel et l'on peut citer notamment, les groupes fonctionnels de type $NR_9R'_9$ dans lesquels $R_9$, $R'_9$, identiques ou différents représentent un atome d'hydrogène, un groupe alkyle, linéaire ou ramifié ayant de 1 à 12 atomes de carbone, un groupe phényle, un groupe benzyle ou un groupe acyle ayant de 2 à 12 atomes de carbone de préférence, un groupe acétyle ou benzoyle.

Comme exemple plus spécifique, on peut mentionner entre autres, l'acide 2-méthyl-2-buténoïque.

Une première classe de substrats auxquels s'appliquent plus préférentiellement le procédé de l'invention sont les acides acryliques substitués précurseurs d'aminoacides et/ou dérivés.

Sous le terme acides acryliques substitués, on entend l'ensemble des composés dont la formule dérive de celle de l'acide acrylique et en substituant au plus deux des atomes d'hydrogène portés par les atomes de carbone éthylénique par un groupe hydrocarboné ou par un groupe fonctionnel.

Ils peuvent être symbolisés par la formule chimique suivante :

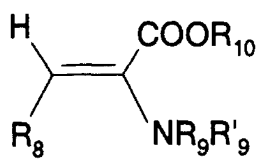

(Va)

dans ladite formule (Va) :

- $R_9$, $R'_9$, identiques ou différents représentent un atome d'hydrogène, un groupe alkyle, linéaire ou ramifié ayant de 1 à 12 atomes de carbone, un groupe phényle ou un groupe acyle ayant de 2 à 12 atomes de carbone de préférence, un groupe acétyle ou benzoyle,
- $R_8$ représente un atome d'hydrogène, un groupe alkyle ayant de 1 à 12 atomes de carbone, un radical cycloalkyle ayant de 3 à 8 atomes de carbone, un radical arylalkyle ayant de 6 à 12 atomes de carbone, un radical aryle ayant de 6 à 12 atomes de carbone, un radical hétérocyclique ayant de 4 à 7 atomes de carbone,
- $R_{10}$ représente un atome d'hydrogène ou un groupe alkyle linéaire ou ramifié, ayant de 1 à 4 atomes de carbone.

Comme exemples plus spécifiques de groupes $R_8$, on peut mentionner un groupe alkyle tel que méthyle, éthyle, isopropyle, isobutyle ; un groupe cycloalkyle tel que cyclopentyle, cyclohexyle ; un groupe aromatique tel que phényle, naphtyle ou un groupe hétérocyclique tel que furyle, pyrannyle, benzopyrannyle, pyrrolyle, pyridyle, indolyle.

Le groupe $R_{10}$ est préférentiellement un atome d'hydrogène.

Parmi les acides acryliques substitués précurseurs d'acides aminés, on peut citer l'acide N-acétyl α-amino β-phénylacrylique, l'acide N-benzoyl α-amino β-phénylacrylique, dans lesquels le noyau phényle est éventuellement substitué par un ou plusieurs groupes alkyle, alcoyloxy ou hydroxy, l'acide N-acétyl α-amino β-indolylacrylique, l'acide N-benzoyl α-amino β-indolylacrylique, l'acide N-acétyl α-amino β-isobutyl acrylique.

On peut citer plus particulièrement :

- l'α-acétamidocinnamate de méthyle,
- l'acétamidoacrylate de méthyle,
- l'acide benzamidocinnamique,
- l'acide α-acétamidocinnamique.

L'invention s'applique également, tout à fait bien pour effectuer l'hydrogénation de l'acide itaconique et/ou dérivé, et plus spécifiquement aux composés répondant à la formule (Vb) :

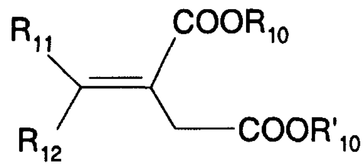
(Vb)

dans ladite formule (Vb) :

- $R_{11}$, $R_{12}$, identiques ou différents représentent un atome d'hydrogène, un groupe alkyle linéaire ou ramifié ayant de 1 à 12 atomes de carbone, un radical cycloalkyle ayant de 3 à 8 atomes de carbone, un radical arylalkyle ayant de 6 à 12 atomes de carbone, un radical aryle ayant de 6 à 12 atomes de carbone, un radical hétérocyclique ayant de 4 à 7 atomes de carbone,
- $R_{10}$, $R'_{10}$, identiques ou différents représentent un atome d'hydrogène ou un groupe alkyle linéaire ou ramifié, ayant de 1 à 4 atomes de carbone.

Les substrats préférés répondent à la formule (Vb) dans laquelle $R_{11}$, $R_{12}$, identiques ou différents représentent un atome d'hydrogène, un groupe alkyle ayant de 1 à 4 atomes de carbone et $R_{10}$, $R'_{10}$, identiques ou différents représentent un atome d'hydrogène ou un groupe méthyle.

Comme exemples plus particuliers, on peut mentionner notamment l'acide itaconique et l'itaconate de diméthyle.

Le procédé de l'invention s'applique tout particulièrement à la préparation des acides arylpropioniques par hydrogénation d'un substrat répondant à la formule (Vc) :

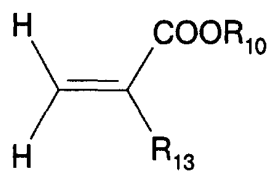
(Vc)

dans ladite formule (Vc) :

- $R_{10}$ représente un atome d'hydrogène ou un groupe alkyle linéaire ou ramifié, ayant de 1 à 4 atomes de carbone,
- $R_{13}$ représente un groupe phényle ou naphtyle, éventuellement porteur d'un substituant ou plusieurs substituants R :
  - R peut représenter $R_0$, l'un des groupes suivants :
    - un groupe alkyle ou alcényle linéaire ou ramifié ayant de 1 à 12 atomes de carbone, de préférence, un groupe alkyle linéaire ou ramifié ayant de 1 à 4 atomes de carbone,
    - un groupe alkoxy linéaire ou ramifié ayant de 1 à 12 atomes de carbone, de préférence, un groupe alkoxy linéaire ou ramifié ayant de 1 à 4 atomes de carbone,
    - un groupe acyloxy linéaire ou ramifié ayant de 2 à 8 atomes de carbone, de préférence, un groupe acétoxy,
    - un groupe acylamido linéaire ou ramifié ayant de 1 à 8 atomes de carbone, de préférence, un groupe acétamido,
    - un groupe $NO_2$,

- R peut représenter $R_0$', l'un des groupes plus complexes suivants :

  . un groupe de formule

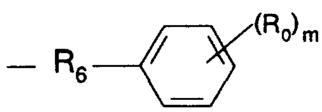

dans lequel :

- $R_6$ représente un lien valentiel ; un groupe hydrocarboné divalent, linéaire ou ramifié, saturé ou insaturé ayant de 1 à 6 atomes de carbone tel que par exemple, méthylène, éthylène, propylène, isopropylène, isopropylidène ou l'un des groupes suivants dénommés Z :
  $-O-$ ; $-CO-$ ; $-COO-$ ; $-NR_7-$; $-CO-NR_7-$ ; $-S-$ ; $-SO_2-$ ; $-NR_7-CO-$;
  dans lesdites formules $R_7$ représente un atome d'hydrogène, un groupe alkyle linéaire ou ramifié ayant de 1 à 6 atomes de carbone,
- $R_0$ a la signification donnée précédemment,
- m est un nombre entier de 0 à 4.

Comme exemples spécifiques, on peut citer l'acide 2-(3-benzoylphényl)propionique (Kétoprofène® ), l'acide 2-(4-isobutylphényl)propionique (Ibuprofène® ), l'acide 2-(5-méthoxynaphtyl)propionique (Naproxène® ).
L'hydrogénation asymétrique sélective desdits substrats est effectuée en utilisant comme catalyseurs les complexes métalliques de l'invention ligandés par les diphosphines optiquement actives de formule générale (Ia) ou (Ib).
Quand les complexes de l'invention diphosphine-métal de transition sont utilisés comme catalyseur d'hydrogénation asymétrique des acides carboxyliques insaturés, le produit désiré peut être obtenu avec un rendement optique élevé.
En choisissant l'un des isomères optiques de la diphosphine ayant un pouvoir rotatoire (+) ou (-), et en utilisant un complexe diphosphine-métal de transition comprenant l'isomère choisi, l'acide carboxylique insaturé est hydrogéné en un composé ayant la configuration absolue désirée, avec un rendement optique élevé.
L'hydrogénation s'effectue généralement à une température comprise entre 20 et 100°C.
La pression d'hydrogène peut être comprise entre 0,1 et 200 bar, et plus préférentiellement entre 1 et 150 bar.
Le complexe diphosphine/métal de transition est utilisé de telle manière que le rapport entre le nombre d'atomes de métal présents dans le complexe et le nombre de moles du composé à hydrogéner soit compris entre 0,1 et 0,0001.
Le procédé d'hydrogénation est mis en oeuvre, de préférence, dans un solvant organique. On fait appel à n'importe quel solvant dans la mesure où il est stable dans les conditions réactionnelles.
On a recours de préférence, à un solvant organique polaire et plus particulièrement aux solvants suivants :

- les éther-oxydes aliphatiques, cycloaliphatiques ou aromatiques et, plus particulièrement, le diéthyléther, le dipropyléther, le diisopropyléther, le dibutyléther, le méthyltertiobutyléther, le ditertiobutyléther, le diméthyléther de l'éthylèneglycol, le diméthyléther du diéthylèneglycol ; le diphényléther, le dibenzyléther, l'anisole, le phénétole, le 1,4-diméthoxybenzène, le vératrole ; le 1,4-dioxane, le tétrahydrofurane (THF),
- les alcools mono- ou polyhydroxylés et plus particulièrement, les monoalcools aliphatiques tels que le méthanol, l'éthanol, le propanol, le butanol, le sec-butanol, le tert-butanol, le pentanol, l'hexanol ; les dialcools aliphatiques tels que l'éthylène glycol, le diéthylène glycol, le propylène glycol ; les alcools cycloaliphatiques tels que le cyclopentanol, le cyclohexanol,
- les cétones aliphatiques telles que l'acétone, la méthyéthylcétone, la diéthylcétone,
- les esters aliphatiques tels que notamment l'acétate de méthyle, l'acétate d'éthyle, l'acétate de propyle.

La concentration du substrat dans le solvant organique varie avantageusement entre 0,01 et 1 mol/l.
On peut éventuellement ajouter, après la formation du complexe d'hydrogénation, un composé basique.
Ce composé basique peut être une base alcaline telle que l'hydroxyde de sodium ou de potassium ou bien une amine primaire, secondaire ou tertiaire, et plus particulièrement, la pyridine, la pipéridine, la triéthylamine et de préférence, la triéthylamine.
La quantité de base ajoutée est telle que le rapport entre le nombre de moles de base et le nombre d'atomes

métalliques présents dans le complexe diphosphine/métal de transition, soit compris entre 0 et 25, de préférence, entre 0 et 12.

On donne ci-après un mode de réalisation préférentiel du procédé de l'invention.

On met en oeuvre ledit procédé dans un autoclave que l'on purge à l'aide d'un gaz inerte, de préférence, l'azote. On charge de préférence, le substrat en solution dans le solvant organique, puis le catalyseur également en solution dans le solvant organique.

On remplace l'azote par l'hydrogène.

L'hydrogénation est terminée lorsque la pression d'hydrogène devient stable.

Le procédé d'hydrogénation selon l'invention permet d'accéder avec des excès énantiomériques élevés, aux différents énantiomères de nombreux dérivés.

Les exemples suivants illustrent la présente invention.

On donne ci-après un exemple de réalisation de la présente invention qui est donné à titre illustratif.

L'exemple 1 concerne la préparation des nouvelles diphosphines optiquement actives de bis-[1-phospha-2,3-diphényl-4,5-diméthyl-norbornadiène] (S,S) - (+) et (R,R) - (-).

Dans les exemples 2 à 4, on décrit la synthèse des catalyseurs utilisés en hydrogénation.

Les exemples 5 à 13 correspondent aux exemples d'application.

EXEMPLES

Exemple 1 :

Phospholyllithium : (III)

Dans un ballon de 250 ml, on introduit 11,3 g (0,06 mol) de 1-phényl-3,4-diméthylphosphole, 0,8 g de lithium métal et 100 ml de tétrahydrofurane distillé.

Le mélange est agité sous argon pendant 2 heures, dans un bain d'eau froide.

La solution devient brune.

L'apparition du phospholyllithium est contrôlée par RMN$^{31}$P.

RMN$^{31}$P = $\delta$(THF) = 55,8 ppm.

Afin de piéger le phényllithium, on ajoute 2,7 g de chlorure d'aluminium à 0°C.

On laisse réagir 30 minutes à 0°C.

1,1' bisphosphole: (IV)

Au mélange précédent, on ajoute à température ambiante goutte à goutte, 6 g (0,025 mol) de diiode en solution dans 25 ml de tétrahydrofurane.

Lorsque 90 % de cette solution sont introduits, on vérifie par RMN$^{31}$P, la disparition de (III).

RMN$^{31}$P = $\delta$(THF) = - 22,4 ppm.

On extrait sous azote le 1,1' bisphosphole (IV) du milieu, à l'aide d'hexane.

Bis-[1-phospha-2,3 diphényl-4,5-diméthylnorbornadiène] : (I m) et (I r)

La solution précédente est évaporée à sec, à l'abri de l'air et portée à 140°C.

On introduit alors 8 g de diphénylacétylène et on laisse réagir pendant 15 à 20 minutes.

La disparition de (IV) est une nouvelle fois suivie par RMN$^{31}$P.

Le spectre est composé de 2 singulets correspondants aux deux diastéréoisomères.

Le produit est extrait à l'éther et lavé à l'eau.

Les phases organiques sont rassemblées puis évaporées à sec.

Le résidu est alors purifié par chromatographie sur colonne de silice (élution à l'hexane pour éliminer le diphénylacétylène en excès puis avec un mélange hexane/dichlorométhane : 80/20 en volume).

Le rendement global est de 30 %.

Complexe de palladium II avec (I m) et (I r) : (VI m) et (VI r)

Dans un ballon de 500 ml, on introduit 5 g (8,25 mmol) de (I m) et de (I r) que l'on dissout dans 200 ml de dichlorométhane.

On ajoute alors goutte à goutte 3 g (8,25 mmol) de $PdCl_2(PhCN)_2$ dans 100 ml de dichlorométhane.

La réaction conduite sous argon, est immédiate.

La solution est évaporée à sec et le résidu est soumis à une chromatographie sur silice afin de séparer les deux diastéréoisomères.
On élue à l'aide de dichlorométhane pour éliminer les impuretés, puis d'un mélange de dichlorométhane et d'acétate d'éthyle : 95/5 en volume pour séparer le racémique et enfin d'un mélange dichlorométhane/acétate d'éthyle : 80/20 en volume pour séparer le méso.
RMN$^{31}$ P = $\delta(CH_2Cl_2)$ = 81,9 ppm - isomère minoritaire correspondant au racémique.
RMN$^{31}$ P = $\delta(CH_2Cl_2)$ = 88,1 ppm - isomère majoritaire correspondant au méso.

Décomplexation de (VI r)

Dans un ballon de 100 ml, on introduit 1,5 g (0,002 mol) de (VI r) racémique et 20 ml de dichlorométhane.
On ajoute alors 0,5 g de cyanure de sodium et quelques millilitres d'eau (3 ml).
On agite vigoureusement sous argon pendant 10 à 15 minutes.
Le bis-[1-phospha-2,3 diphényl-4,5-diméthylnorbornadiène] (I r) est alors extrait avec du dichlorométhane.
La phase organique est lavée à l'eau puis séchée sur sulfate de sodium.
On récupère ainsi (I r) pur.
Le rendement global de la séparation des diastéréoisomères est de 90 %.
La caractérisation du mélange racémique (I r) est la suivante :

RMN$^{31}$P = $\delta(CDCl_3)$ = - 13,2 ppm.

RMN$^{1}$H = $\delta(CDCl_3)$ = 1,31(s, 6H, $CH_3$) ; 1,69(s, 6H, $CH_3$) ; 2,02-2,20 (m, 4H, $CH_2$ pont) ; 6,86-7,29 (m, 20H, phényls).

Complexe binucléaire de palladium II :

Dans 12 ml de benzène, on introduit sous azote 290 mg (0,5 mmol) de (I r) racémique et 300 mg (0,5 mmol) de (+)-di-$\mu$-chloro-bis[(S)-N,N,-diméthyl-$\alpha$-phényléthylamine-2C,N]-dipalladium II.
La complexation est rapide et suivie par RMN$^{31}$P.
La solution brune est évaporée à sec et le résidu chromatographié pour séparer les deux diastéréoisomères (élution toluène/acétate d'éthyle : 80/20 en volume).
On récupère ainsi les deux énantiomères isolés purs sous forme de deux complexes diastéréoisomères de formule :

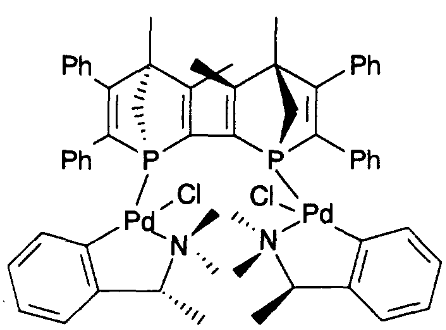

et

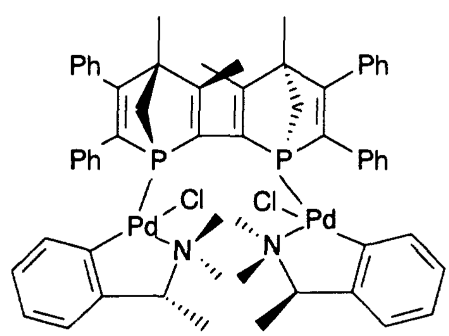

Ces énantiomères sont recouvrés purs en décomplexant comme pour (VI r).
On identifie les diphosphines de formules (Ia) et (Ib) respectivement comme suit:

RMN$^{31}$P = $\delta(CDCl_3)$ = - 13,2 ppm - $[\alpha]_D$ = + 231° (c = 1, $C_6D_6$).

RMN$^{31}$P = $\delta(CDCl_3)$ = - 13,2 ppm - $[\alpha]_D$ = - 198° (c = 1, $C_6D_6$)

(avec un $[\alpha]_D$ déterminé pour une concentration de 10 mg/ml et à température ambiante).

Exemple 2 :

Dans cet exemple, on décrit la préparation d'un complexe de formule $[Rh^+(COD)(P^*P)]PF_6^-$ dans laquelle COD représente le 1,5-cyclooctadiène et (P*P) représente la diphosphine de formule (Ib).
Dans un schlenk de 10 ml, on dissout, sous argon, dans 3 ml d'acétone, 11,6 mg de $Rh(COD)_2PF_6$.
On ajoute alors goutte à goutte, toujours sous gaz inerte, une solution de 7,5 mg de ladite diphosphine dans l'acétone.

Après quelques minutes d'agitation, on obtient le complexe attendu.
RMN $^{31}P$ : $\delta$ = 73,8 ppm, J(Rh-P) = 155Hz.

Exemple 3 :

Dans cet exemple, on décrit la préparation d'un complexe de formule $[Rh^+(COD)(P*P)]PF_6^-$ dans laquelle COD représente le 1,5-cyclooctadiène et (P*P) représente la diphosphine de formule (Ia).
La préparation dudit complexe est effectuée selon le même mode opératoire que celui de l'exemple 2.

Exemple 4 :

Dans cet exemple, on décrit la préparation d'un complexe de formule $RuBr_2(P^*P)$ dans laquelle (P*P) représente la diphosphine de formule (Ia).
Dans un schlenk de 10 ml, on dissout sous argon, dans 2 ml d'acétone, 7,5 mg de diphosphine, 4 mg de Ru $(COD)(allyl)_2$.
On ajoute ensuite, goutte à goutte, 0,11 ml d'une solution aqueuse d'acide bromhydrique 0,29 M dans du méthanol.
On agite 30 mn à température ambiante (~ 20°C) et on obtient le complexe attendu.
RMN $^{31}P$ : système AB $\delta$ = 98,2 ppm, 88,1 ppm ($J_{AB}$ = 21 Hz).

Exemple 5 :

Dans cet exemple, on effectue l'hydrogénation asymétrique, à l'aide du catalyseur de l'exemple 2, du composé suivant :

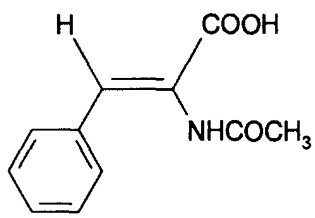

Dans un ballon, on dissout 400 mg dudit composé dans 20 ml de méthanol.
On prépare ensuite comme proposé plus haut, le complexe de l'exemple 2.
On évapore l'acétone et on dissout le résidu dans 5 ml de méthanol.
On introduit alors les 2 solutions dans un autoclave préalablement purgé et maintenu sous atmosphère d'azote.
Puis, on introduit l'hydrogène jusqu'à une pression de 3 atmosphères.
On agite à 20°C pendant 1 h.
On vide l'hydrogène en excès et on récupère la solution de la réaction.
Le solvant est évaporé et le résidu analysé en RMN$^1$H pour vérifier l'avancement de la réaction.
La réaction est quantitative.
L'excès énantiomérique est déterminé par chromatographie liquide haute performance chirale (colonne chirale protéine HSA 150 x 6,4 mm Shandon® ) et la configuration absolue du produit par mesure de l'$[\alpha]_D$, par polarimétrie.
Avec la diphosphine (Ib), ee ≥ 98 %, $[\alpha]_D$ (éthanol, c = 1) > 0.

Exemple 6:

Dans cet exemple, on effectue l'hydrogénation asymétrique, à l'aide du catalyseur de l'exemple 4, du composé suivant :

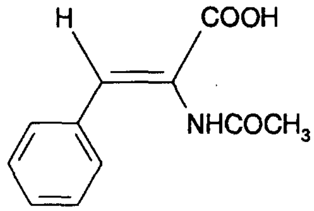

La mise en oeuvre est la même qu'avec 1. La différence réside dans le catalyseur, le temps de réaction est inférieur à 24 heures et la pression atmosphérique.
Avec la diphosphine (Ia), ee = 80 %, $[\alpha]_D$ (éthanol, c = 1) < 0.

Exemple 7 :

Dans cet exemple, on effectue l'hydrogénation asymétrique, à l'aide du catalyseur de l'exemple 3. du composé suivant :

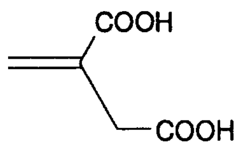

La mise en oeuvre est identique.
On introduit 165 mg d'acide itaconique pour toujours les mêmes quantités de solvant et de catalyseur.
La réaction s'effectue à température ambiante, en moins de 3 heures.
Avec la diphosphine (Ia), ee = 80 %, $[\alpha]_D$ (éthanol, c = 2,16) < 0.

Exemple 8 :

Dans cet exemple, on effectue l'hydrogénation asymétrique, à l'aide du catalvseur de l'exemple 2, du composé suivant :

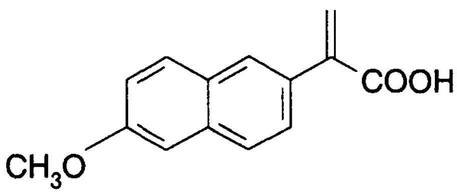

Dans une ampoule, on dissout 245 mg dudit composé dans 8 ml de méthanol.
On prépare ensuite, comme proposé plus haut, le complexe de l'exemple 2.
On évapore l'acétone et on dissout le résidu dans 2 ml de méthanol.
On introduit alors cette solution dans l'ampoule, qui est elle-même placée dans l'autoclave préalablement purgé et maintenu sous atmosphère d'argon.
On introduit alors l'hydrogène jusqu'à une pression de 4 atmosphères.
On agite à 20°C pendant 2 heures.
On vide l'hydrogène en excès et on récupère la solution de la réaction.
La solution de la réaction est évaporée et le résidu analysé en $RMN^1H$ pour vérifier l'avancement de la réaction.
L'avancement est de 30 %.
L'excès énantiomérique (ee) est déterminé par chromatographie liquide haute performance chirale (colonne Chiralpak -AD® ).
Avec la disphosphine (Ib), ee > 98 %.

Exemple 9 :

Dans cet exemple, on décrit la préparation d'un complexe de formule $Ru(OAc)_2(P^*P)$ dans laquelle OAc représente un groupement acétate et (P*P) la diphosphine de formule (Ib).

Dans un schlenk de 10 ml, on dissout sous argon 10 mg de diphosphine (Ib) et 6 mg de Ru $(Me\text{-}allyl)_2(COD)$ dans 2 ml d'acétone.

On ajoute alors goutte à goutte une solution de 4 mg de $CCl_3CO_2H$ dans 1 ml de méthanol.

Après quelques minutes d'agitation, on ajoute un large excès d'acétate de sodium dissout dans 1 ml de méthanol et on obtient en quelques minutes d'agitation le complexe attendu.

RMN$^{31}$P : $\delta$ = 107,9 ppm

Exemple 10:

Dans cet exemple, on décrit la préparation d'un complexe de formule $Ru(OAc)_2(P^*P)$ dans laquelle OAc représente un groupement acétate et (P*P) la diphosphine de formule (Ia).

Ledit complexe est préparé selon le mode opératoire de l'exemple 9.

Exemple 11 :

Dans cet exemple, on effectue l'hydrogénation asymétrique, à l'aide du catalyseur de l'exemple 9, du composé suivant :

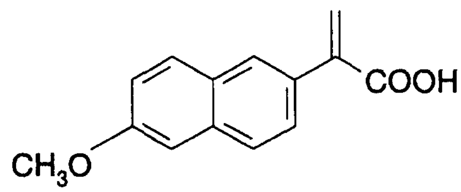

La mise en oeuvre est la même que dans l'exemple 8. La différence réside dans le catalyseur, le temps de réaction qui est de 12 heures et la pression de 130 atmosphères. L'avancement est de 12 %.

Avec la diphosphine (Ib), ee > 95 %.

Exemple 12 :

Dans cet exemple, on effectue l'hydrogénation asymétrique, à l'aide du catalyseur de l'exemple 10, du composé suivant :

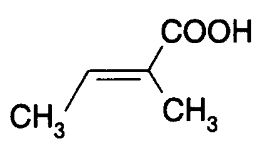

Dans un ballon, on dissout 100 mg dudit composé dans 4 ml de méthanol.

On prépare ensuite comme proposé plus haut, le complexe de l'exemple 9.

On introduit alors les deux solutions dans un autoclave préalablement purgé et maintenu sous atmosphère d'azote.

On introduit alors l'hydrogène jusqu'à une pression de 4 atmosphères.

On agite à 20°C pendant 3 heures.

On vide l'hydrogène en excès et on récupère la solution de la réaction.

La solution est évaporée et le résidu analysé en RMN$^1$H pour vérifier l'avancement de la réaction.

L'avancement est de 90 %.

L'excès énantiomérique (ee) est déterminé par chromatographie liquide haute performance chirale (colonne Chiralcel -OJ-R® ).

Avec la diphosphine (Ia), ee = 57 %.

Exemple 13 :

Dans cet exemple, on effectue l'hydrogénation asymétrique, à l'aide du catalyseur de l'exemple 2, du composé suivant :

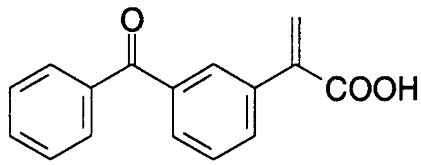

Dans une ampoule, on dissout 270 mg dudit composé dans 8 ml de méthanol.
On prépare ensuite, comme proposé plus haut, le complexe 1 de l'exemple 2.
On évapore l'acétone et on dissout le résidu dans 3 ml de méthanol.
On introduit alors cette solution dans l'ampoule, qui est elle-même placée dans l'autoclave préalablement purgé et maintenu sous atmosphère d'argon.
On introduit alors l'hydrogène jusqu'à une pression de 5 atmosphères.
On agite à 20°C pendant 2 heures.
On vide l'hydrogène en excès et on récupère la solution de la réaction.
La solution de la réaction est évaporée et le résidu analysé en RMN$^1$H pour vérifier l'avancement de la réaction.
L'avancement est de 70 %.
L'excès énantiomérique (ee) est déterminé par chromatographie liquide haute performance chirale (colonne Chiralcel -OJ-R® ).
Avec la disphosphine (Ib), ee > 98 %.

## Revendications

1. Diphosphines optiquement actives de bis-[1-phospha-2,3-diphényl-4,5-diméthylnorbornadiène] répondant aux formules suivantes :

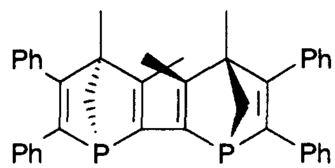

(Ia) - (S,S) - (+)

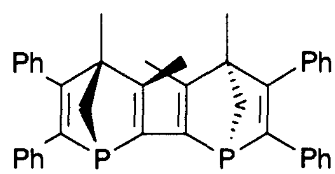

(Ib) - (R,R) - (-)

2. Procédé de dédoublement du mélange racémique de bis-[1-phospha-2,3-diphényl-4,5-diméthylnorbornadiène] qui consiste à le faire réagir avec un complexe de palladium et/ou de platine comme auxiliaire chiral, dans un solvant organique formant ainsi des complexes diastéréoisomères, puis à dédoubler lesdits complexes optiquement purs.

3. Procédé selon la revendication 2 caractérisé par le fait que l'auxiliaire chiral répond à la formule générale (VII) :

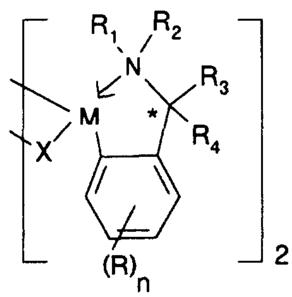

(VII)

dans ladite formule :

- M représente le palladium et/ou le platine,
- $R_1$, $R_2$, $R_3$ et $R_4$ représentent un atome d'hydrogène ou un radical alkyle ayant de 1 à 10 atomes de carbone ou un radical cycloalkyle ayant de 3 à 10 atomes de carbone,
- $R_3$ et $R_4$ sont différents et au moins l'un des deux représente un atome d'hydrogène,
- R a la signification donnée pour $R_1$, $R_2$, $R_3$ et $R_4$,
- X représente un atome d'halogène,
- n est un nombre de 0 à 4,
- lorsque n est supérieur à 1, deux radicaux R et les 2 atomes successifs du cycle benzénique peuvent former entre eux, un cycle ayant de 5 à 7 atomes de carbone.

**4.** Procédé selon l'une des revendications 2 et 3 caractérisé par le fait que l'auxiliaire chiral répond à la formule générale (VII) dans laquelle $R_1$, $R_2$, $R_3$ et $R_4$ représentent un atome d'hydrogène ou un radical méthyle, X représente un atome de chlore et n est égal à 0.

**5.** Procédé selon l'une des revendications 2 et 3 caractérisé par le fait que l'auxiliaire chiral répond à la formule générale (VII) dans laquelle $R_1$, $R_2$, $R_3$ et $R_4$ représentent un atome d'hydrogène ou un radical méthyle, X représente un atome de chlore et lorsque n est égal à 2, deux radicaux R forment un cycle benzénique.

**6.** Procédé selon l'une des revendications 2 à 4 caractérisé par le fait que l'auxiliaire chiral répond à la formule générale (VII') :

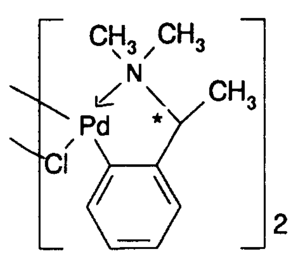

(VII')

**7.** Procédé selon l'une des revendications 2 à 6 caractérisé par le fait que la quantité de complexe des métaux précités exprimée en métal est de 0,5 à 1 atome de métal par atome de phosphore.

**8.** Procédé selon l'une des revendications 2 à 7 caractérisé par le fait que le solvant organique est choisi parmi :

- les hydrocarbures aliphatiques et plus particulièrement les paraffines tels que notamment, le pentane, l'hexane, l'heptane, l'octane, l'isooctane, le nonane, le décane, l'undécane, le tétradécane, l'éther de pétrole et le cyclohexane ; les hydrocarbures aromatiques comme notamment le benzène, le toluène, les xylènes, l'éthylbenzène, les diéthylbenzènes, les triméthylbenzènes, le cumène, le pseudocumène, les coupes pétrolières constituées d'un mélange d'alkylbenzènes notamment les coupes de type Solvesso®,
- les hydrocarbures halogénés aliphatiques ou aromatiques, et l'on peut mentionner : les hydrocarbures perchlorés tels que notamment le trichlorométhane, le tétrachloroéthylène ; les hydrocarbures partiellement chlorés tels que le dichlorométhane, le dichloroéthane, le tétrachloroéthane, le trichloroéthylène, le 1-chlorobutane, le 1,2-dichlorobutane ; le monochlorobenzène, le 1,2-dichlorobenzène, le 1,3-dichlorobenzène, le 1,4-dichlorobenzène ou des mélanges de différents chlorobenzènes.

**9.** Procédé selon la revendication 8 caractérisé par le fait que le solvant organique est le benzène ou le toluène.

**10.** Procédé selon l'une des revendications 2 à 9 caractérisé par le fait que la concentration de la diphosphine dans le solvant réctionnel est de préférence, entre 0,05 et 1 mole/litre et encore plus particulièrement entre 0,05 et 0,2 mole/litre.

**11.** Procédé selon l'une des revendications 2 à 10 caractérisé par le fait que la séparation est conduite à température ambiante généralement sous atmosphère contrôlée de gaz inertes, de préférence, sous azote.

**12.** Procédé selon l'une des revendications 2 à 11 caractérisé par le fait que l'on effectue la séparation des deux énantiomères par chromatographie liquide sur colonne, avec, de préférence un support en silice.

**13.** Procédé selon la revendication 12 caractérisé par le fait que l'on élue la colonne avec un mélange de solvants appropriés, de préférence, un mélange toluène/acétate d'éthyle comprenant préférentiellement, 80 % en volume de toluène et 20 % en volume d'acétate d'éthyle.

**14.** Procédé selon la revendication 13 caractérisé par le fait que l'on récupère les deux énantiomères de la diphosphine purs en effectuant la solubilisation des complexes dans un solvant organique tel que, par exemple, le dichlorométhane, puis la décomplexation à l'aide d'un sel de l'acide cyanhydrique, de préférence, un sel alcalin et encore plus préférentiellement le sodium.

**15.** Complexe comprenant une diphosphine optiquement active et un auxiliaire chiral caractérisé par le fait qu'il répond à l'une des formules suivantes :

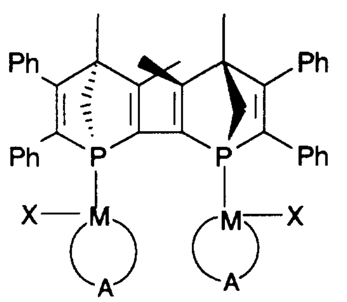

(VIII a)

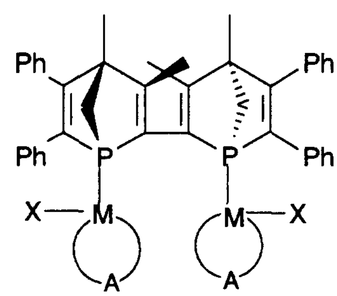

(VIII b)

dans lesdites formules, M représente le palladium ou le platine, X un atome d'halogène, de préférence, le chlore et A symbolise le reste d'un complexe métallique chiral répondant à l'une des formules (VII) et préférentiellement (VII') selon l'une des revendications 3 à 6.

**16.** Complexe comprenant une diphosphine optiquement active et un métal de transition caractérisé par le fait que le ligand répond à l'une des formules suivantes :

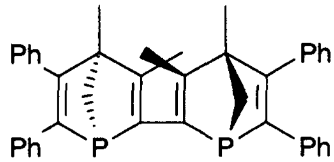

(Ia) - (S,S) - (+)

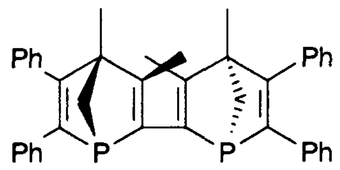

(Ib) - (R,R) - (-)

**17.** Complexe selon la revendication 16 caractérisé par le fait que le métal de transition est choisi parmi : le rhodium, le ruthénium, le rhénium, l'iridium, le cobalt, le nickel, le platine, le palladium.

**18.** Complexe comprenant une diphosphine optiquement active et du rhodium et/ou iridium selon l'une des revendications 16 et 17 caractérisé par le fait qu'il est représenté par les formules suivantes :

$$[M\ L_2(P^*P)]\ Y \qquad (IIa)$$

$$[M\ L_2(P^*P)]\ Y \qquad (IIb)$$

dans lesdites formules :

- (P*P) représente dans la formule (IIa) la diphosphine de formule (Ia) et dans la formule (IIb) la diphosphine de formule (Ib),
- M représente le rhodium ou l'iridium,
- Y représente un ligand anionique coordinant,
- L représente un ligand neutre.

**19.** Complexe selon la revendication 18 caractérisé par le fait qu'il répond à la formule (IIa) ou (IIb) dans laquelle :

- L représente une oléfine ayant de 2 à 12 atomes de carbone et deux ligands L peuvent être liés entre eux pour former une chaîne hydrocarbonée polyinsaturée, linéaire ou cyclique ; L représentant de préférence, le 1,5-cyclooctadiène, le norbornadiène, l'éthylène,
- Y représente un anion $PF_6^-$, $PCl_6^-$, $BF_4^-$, $BCl_4^-$, $SbF_6^-$, $SbCl_6^-$, $BPh_4^-$, $ClO_4^-$, CN-, $CF_3SO_3^-$, halogène de préférence, $Cl^-$ ou $Br^-$, un anion 1,3-dicétonate, alkylcarboxylate, haloalkylcarboxylate avec un radical alkyle inférieure, un anion phénylcarboxylate ou phénolate dont le cycle benzénique peut être substitué par des radicaux alkyle inférieurs et/ou des atomes d'halogène.

**20.** Complexe comprenant une diphosphine optiquement active et de l'iridium selon l'une des revendications 16 et 17 caractérisé par le fait qu'il est représenté par les formules suivantes :

$$[Ir\ L\ (P^*P)]\ Y \qquad (IIIa)$$

$$[Ir\ L\ (P^*P)]\ Y \qquad (IIIb)$$

dans lesdites formules, (P*P), L et Y ont les significations données pour les formules (IIa) et (IIb).

**21.** Complexe comprenant une diphosphine optiquement active et du ruthénium selon l'une des revendications 16 et 17 caractérisé par le fait qu'il est représenté par les formules suivantes :

$$[RuY_1Y_2(P^*P)] \qquad (IVa)$$

$$[RuY_1Y_2(P^*P)] \qquad (IVb)$$

dans lesdites formules :

- (P*P) représente dans la formule (IVa) la diphosphine de formule (Ia) et dans la formule (IVb) la diphosphine de formule (Ib),
- $Y_1$ et $Y_2$, identiques ou différents, représentent de préférence, un anion $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$, un atome d'halogène, plus particulièrement, chlore ou brome ou un anion carboxylate, préférentiellement, acétate, trifluoroacétate.

**22.** Complexe comprenant une diphosphine optiquement active et du ruthénium selon l'une des revendications 16 et 17 caractérisé par le fait qu'il est représenté par les formules suivantes :

$$[RuY_1Ar(P^*P)Y_2] \qquad (IVc)$$

$$[RuY_1Ar(P^*P)Y_2] \qquad (IVd)$$

dans lesdites formules :

- (P*P) représente dans la formule (IVc) la diphosphine de formule (Ia) et dans la formule (IVd) la diphosphine de formule (Ib),
- Ar représente le benzène, le p-méthylisopropylbenzène, l'hexaméthylbenzène,
- $Y_1$ représente un atome d'halogène, de préférence, chlore ou brome, -$Y_2$ représente un anion de préférence, $PF_6$-, $PCl_6$-, $BF_4$-, $BCl_4$-, $SbF_6$-, $SbCl_6$-, $BPh_4$-, $ClO_4$-, $CF_3SO_3$-.

**23.** Procédé de préparation d'un complexe selon l'une des revendications 16 à 22 caractérisé par le fait qu'il consiste à faire réagir une diphosphine de formule (Ia) ou (Ib) avec le composé de métal de transition, dans un solvant organique approprié.

**24.** Utilisation d'un complexe décrit dans l'une des revendications 16 à 22 en catalyse asymétrique.

**25.** Utilisation d'un complexe décrit dans l'une des revendications 16 à 22 en hydrogénation asymétrique.

**26.** Procédé de préparation d'un acide carboxylique et/ou dérivé, optiquement actif caractérisé par le fait que l'on effectue l'hydrogénation asymétrique d'un acide carboxylique $\alpha,\beta$-insaturé et/ou de ses dérivés en présence d'une quantité efficace d'un complexe métallique comprenant à titre de ligand, la diphosphine optiquement active de formule (Ia) ou (Ib) et un métal de transition.

**27.** Procédé selon la revendication 26 caractérisé par le fait que l'acide carboxylique $\alpha,\beta$-insaturé et/ou dérivé répond à la formule générale suivante :

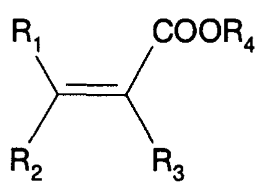

(V)

dans ladite formule (V) :

- $R_1$, $R_2$, $R_3$ et $R_4$, représentent un atome d'hydrogène ou n'importe quel groupe hydrocarboné, dans la mesure où :

. si $R_1$ est différent de $R_2$ et différent d'un atome d'hydrogène alors $R_3$ peut être n'importe quel groupe hydrocarboné ou fonctionnel désigné par $\mathcal{R}$ ,

. si $R_1$ ou $R_2$ représente un atome d'hydrogène et si $R_1$ est différent de $R_2$, alors $R_3$ est différent d'un atome d'hydrogène et différent de $-COOR_4$,

. si $R_1$ est identique à $R_2$ et représente n'importe quel groupe hydrocarboné ou fonctionnel désigné par $\mathcal{R}$ , alors $R_3$ est différent de $-CH-(\mathcal{R})_2$ et différent de $-COOR_4$,

- l'un des groupes $R_1$, $R_2$ et $R_3$ pouvant représenter un groupe fonctionnel.

**28.** Procédé selon la revendication 27 caractérisé par le fait que l'acide carboxylique α,β-insaturé et/ou dérivé répond à la formule (V) dans laquelle les radicaux $R_1$ à $R_4$ identiques ou différents représentent un radical hydrocarboné éventuellement substitué ayant de 1 à 20 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique ; un radical aliphatique saturé ou insaturé, linéaire ou ramifié, porteur d'un substituant cyclique.

**29.** Procédé selon la revendication 26 caractérisé par le fait que l'acide carboxylique mis en oeuvre est un acide acrylique substitué précurseur d'un aminoacide.

**30.** Procédé selon la revendication 29 caractérisé par le fait que l'acide carboxylique mis en oeuvre est un acide acrylique substitué précurseur d'un aminoacide et/ou dérivé répondant à la formule suivante :

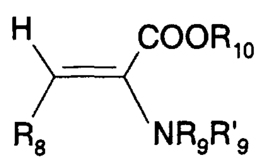
(Va)

dans ladite formule (Va):

- $R_9$, $R'_9$, identiques ou différents représentent un atome d'hydrogène, un groupe alkyle, linéaire ou ramifié ayant de 1 à 12 atomes de carbone, un groupe phényle ou un groupe acyle ayant de 2 à 12 atomes de carbone de préférence, un groupe acétyle ou benzoyle,
- $R_8$ représente un atome d'hydrogène, un groupe alkyle ayant de 1 à 12 atomes de carbone, un radical cycloalkyle ayant de 3 à 8 atomes de carbone, un radical arylalkyle ayant de 6 à 12 atomes de carbone, un radical aryle ayant de 6 à 12 atomes de carbone, un radical hétérocyclique ayant de 4 à 7 atomes de carbone,
- $R_{10}$ représente un atome d'hydrogène ou un groupe alkyle linéaire ou ramifié, ayant de 1 à 4 atomes de carbone.

**31.** Procédé selon la revendication 29 et 30 caractérisé par le fait que l'acide carboxylique mis en oeuvre est l'acide N-acétyl α-amino β-phénylacrylique, l'acide N-benzoyl α-amino β-phénylacrylique, dans lesquels le noyau phényle est éventuellement substitué par un ou plusieurs groupes alkyle, alcoyloxy ou hydroxy, l'acide N-acétyl α-amino β-indolylacrylique, l'acide N-benzoyl α-amino β-indolylacrylique, l'acide N-acétyl α-amino β-isobutyl acrylique.

**32.** Procédé selon la revendication 26 caractérisé par le fait que l'acide carboxylique mis en oeuvre est l'acide itaconique et/ou dérivé répondant à la formule suivante :

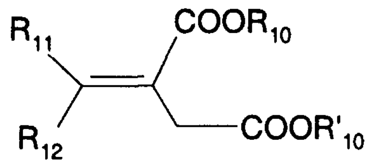
(Vb)

dans ladite formule (Vb) :

- $R_{11}$, $R_{12}$, identiques ou différents représentent un atome d'hydrogène, un groupe alkyle linéaire ou ramifié ayant de 1 à 12 atomes de carbone, un radical cycloalkyle ayant de 3 à 8 atomes de carbone, un radical arylalkyle ayant de 6 à 12 atomes de carbone, un radical aryle ayant de 6 à 12 atomes de carbone, un radical hétérocyclique ayant de 4 à 7 atomes de carbone,
- $R_{10}$, $R'_{10}$, identiques ou différents représentent un atome d'hydrogène ou un groupe alkyle linéaire ou ramifié, ayant de 1 à 4 atomes de carbone.

**33.** Procédé selon la revendication 32 caractérisé par le fait que l'acide carboxylique mis en oeuvre est l'acide itaconique ou l'itaconate de diméthyle.

**34.** Procédé selon la revendication 26 caractérisé par le fait que l'acide carboxylique mis en oeuvre est un substrat répondant à la formule suivante :

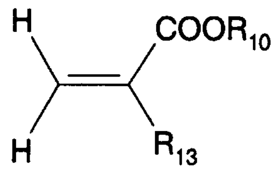

(Vc)

dans ladite formule (Vc) :

- $R_{10}$ représente un atome d'hydrogène ou un groupe alkyle linéaire ou ramifié, ayant de 1 à 4 atomes de carbone,
- $R_{13}$ représente un groupe phényle ou naphtyle, éventuellement porteur d'un substituant ou plusieurs substituants R :

  - R peut représenter $R_0$, l'un des groupes suivants :

    - un groupe alkyle ou alcényle linéaire ou ramifié ayant de 1 à 12 atomes de carbone, de préférence, un groupe alkyle linéaire ou ramifié ayant de 1 à 4 atomes de carbone,
    - un groupe alkoxy linéaire ou ramifié ayant de 1 à 12 atomes de carbone, de préférence, un groupe alkoxy linéaire ou ramifié ayant de 1 à 4 atomes de carbone,
    - un groupe acyloxy linéaire ou ramifié ayant de 2 à 8 atomes de carbone, de préférence, un groupe acétoxy,
    - un groupe acylamido linéaire ou ramifié ayant de 1 à 8 atomes de carbone, de préférence, un groupe acétamido,
    - un groupe $NO_2$,

- R peut représenter $R_0'$, l'un des groupes plus complexes suivants :

  . un groupe de formule

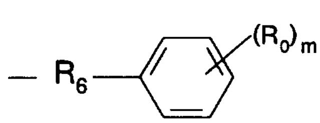

  dans lequel :

  - $R_6$ représente un lien valentiel ; un groupe hydrocarboné divalent, linéaire ou ramifié, saturé ou insaturé ayant de 1 à 6 atomes de carbone tel que par exemple, méthylène, éthylène, propylène, isopropylène, isopropylidène ou l'un des groupes suivants dénommés Z :
    -O- ; -CO- ; -COO- ; $-NR_7-$; $-CO-NR_7-$ ; -S- ; $-SO_2-$ ; $-NR_7-CO-$;

  dans lesdites formules $R_7$ représente un atome d'hydrogène, un groupe alkyle linéaire ou ramifié ayant

de 1 à 6 atomes de carbone,

- $R_0$ a la signification donnée précédemment,
- m est un nombre entier de 0 à 4.

**35.** Procédé selon la revendication 34 caractérisé par le fait que l'acide carboxylique mis en oeuvre est l'acide 2-(3-benzoylphényl)propionique, l'acide 2-(4-isobutylphényl)propionique, l'acide 2-(5-méthoxynaphtyl)propionique.

**36.** Procédé selon l'une des revendications 26 à 35 caractérisé par le fait que l'hydrogénation s'effectue généralement à une température comprise entre 20 et 100°C.

**37.** Procédé selon l'une des revendications 26 à 36 caractérisé par le fait que la pression d'hydrogène peut être comprise entre 0,1 et 200 bar, et plus préférentiellement entre 1 et 150 bar.

**38.** Procédé selon l'une des revendications 26 à 37 caractérisé par le fait que l'on met en oeuvre au moins un complexe selon l'une des revendications 16 à 22.

**39.** Procédé selon l'une des revendications 26 à 38 caractérisé par le fait que le rapport entre le nombre d'atomes de métal présent dans le complexe et le nombre de moles du composé à hydrogéner est compris entre 0,1 et 0,0001.

**40.** Procédé selon l'une des revendications 26 à 39 caractérisé par le fait que l'on ajoute, après la formation du complexe d'hydrogénation, un composé basique.

**Patentansprüche**

**1.** Optisch aktive Diphosphine von Bis-[1-phospha-2,3-diphenyl-4,5-dimethylnorbornadien], die den folgenden Formeln entsprechen:

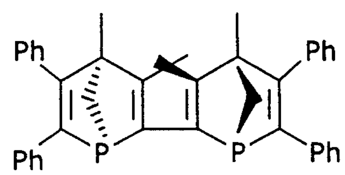

(Ia) - (S,S) - (+)

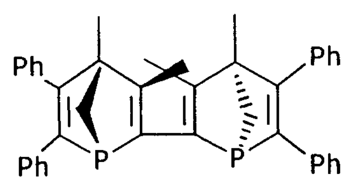

(Ib) - (R,R) - (-)

**2.** Verfahren zur Spaltung des racemischen Gemisches von Bis-[1-phospho-2,3-diphenyl-4,5-dimethylnorbornadien], das darin besteht, es mit einem Palladium- und/oder Platinkomplex als chirales Hilfsmittel in einem organischen Lösungsmittel reagieren zu lassen, um so diastereoisomere Komplexe zu bilden, dann die genannten optisch reinen Komplexe zu spalten.

**3.** Verfahren nach Anspruch 2, dadurch gekennzeichnet, daß das chirale Hilfsmittel der allgemeinen Formel (VII) entspricht:

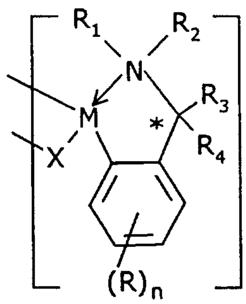

(VII)

wobei in der genannten Formel:

- M Palladium und/oder Platin bedeutet,
- $R_1$, $R_2$, $R_3$ und $R_4$ ein Wasserstoffatom oder einen Alkylrest mit 1 bis 10 Kohlenstoffatomen oder einen Cycloalkylrest mit 3 bis 10 Kohlenstoffatomen bedeuten,
- $R_3$ und $R_4$ verschieden sind und mindestens einer der beiden ein Wasserstoffatom bedeutet,
- R die Bedeutung hat, die $R_1$, $R_2$, $R_3$ und $R_4$ haben,
- X ein Halogenatom bedeutet,
- n eine Zahl von 0 bis 4 ist,
- wenn n größer als 1 ist, zwei Reste R und die 2 aufeinanderfolgenden Atome des Benzolrings miteinander einen Ring mit 5 bis 7 Kohlenstoffatomen bilden können.

**4.** Verfahren nach Anspruch 2 oder 3, dadurch gekennzeichnet, daß das chirale Hilfsmittel der allgemeinen Formel (VII) entspricht, in der $R_1$, $R_2$, $R_3$ und $R_4$ ein Wasserstoffatom oder einen Methylrest bedeuten, X ein Chloratom bedeutet und n gleich 0 ist.

**5.** Verfahren nach Anspruch 2 oder 3, dadurch gekennzeichnet, daß das chirale Hilfsmittel der allgemeinen Formel (VII) entspricht, in der $R_1$, $R_2$, $R_3$ und $R_4$ ein Wasserstoffatom oder einen Methylrest bedeuten, X ein Chloratom bedeutet und, wenn n gleich 2 ist, zwei Reste R einen Benzolring bilden.

**6.** Verfahren nach einem der Ansprüche 2 bis 4, dadurch gekennzeichnet, daß das chirale Hilfsmittel der allgemeinen Formel (VII') entspricht:

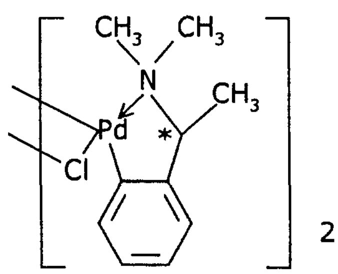

(VII')

**7.** Verfahren nach einem der Ansprüche 2 bis 6, dadurch gekennzeichnet, daß die Menge des Komplexes aus den genannten Metallen, definiert als Metall, von 0,5 bis 1 Metallatome pro Phosphoratome reicht.

**8.** Verfahren nach einem der Ansprüche 2 bis 7, dadurch gekennzeichnet, daß das organische Lösungsmittel gewählt ist aus:

- den aliphatischen Kohlenwasserstoffen und noch spezieller den Paraffinen wie insbesondere Pentan, Hexan, Heptan, Octan, Isooctan, Nonan, Decan, Undecan, Tetradecan, Petrolether und Cyclohexan; den aromatischen Kohlenwasserstoffen wie insbesondere Benzol, Toluol, den Xylolen, Ethylbenzol, den Diethylbenzolen, den Trimethylbenzolen, Cumol, Pseudocumol, den Erdölfraktionen, die aus einem Alkylbenzolgemisch bestehen, insbesondere den Fraktionen des Typs Solvesso® ,

- den aliphatischen oder aromatischen halogenierten Kohlenwasserstoffen und aus denen man erwähnen kann: die perchlorierten Kohlenwasserstoffe wie insbesondere Trichlormethan, Tetrachlorethylen; die teilweise chlorierten Kohlenwasserstoffe wie Dichlormethan, Dichlorethan, Tetrachlorethan, Trichlorethylen, 1-Chlorbutan, 1,2-Dichlorbutan; Monochlorbenzol, 1,2-Dichlorbenzol, 1,3-Dichlorbenzol, 1,4-Dichlorbenzol oder Gemische aus verschiedenen Chlorbenzolen.

**9.** Verfahren nach Anspruch 8, dadurch gekennzeichnet, daß das organische Lösungsmittel Benzol oder Toluol ist.

**10.** Verfahren nach einem der Ansprüche 2 bis 9, dadurch gekennzeichnet, daß die Konzentration des Diphosphins in dem Reaktionslösungsmittel vorzugsweise zwischen 0,05 und 1 mol/Liter und noch spezieller zwischen 0,05 und 0,2 mol/Liter liegt.

**11.** Verfahren nach einem der Ansprüche 2 bis 10, dadurch gekennzeichnet, daß die Trennung bei Umgebungstemperatur im allgemeinen unter kontrollierter Atmosphäre eines inerten Gases, vorzugsweise unter Stickstoff, durchgeführt wird.

**12.** Verfahren nach einem der Ansprüche 2 bis 11, dadurch gekennzeichnet, daß man die Trennung der zwei Enantiomeren durch Flüssig-Säulen-Chromatographie mit vorzugsweise einem Kieselsäureträger durchführt.

**13.** Verfahren nach Anspruch 12, dadurch gekennzeichnet, daß man die Säule mit einem Gemisch aus geeigneten Lösungsmitteln, vorzugsweise einem Toluol/Essigsäureethylester mit vorzugsweise 80 Vol.-% Toluol und 20 Vol.-% Essigsäureethylester, eluiert.

**14.** Verfahren nach Anspruch 13, dadurch gekennzeichnet, daß man die zwei Enantiomeren aus dem reinen Diphosphin gewinnt, indem man die Solubilisation der Komplexe in einem organischen Lösungsmittel, wie z.B. Dichlormethan, und dann die Dekomplexierung mit einem Salz der Blausäure, vorzugsweise einem alkalischen Salz und noch spezieller Natrium, durchführt.

**15.** Komplex mit einem optisch aktiven Diphosphin und einem chiralen Hilfsmittel, dadurch gekennzeichnet, daß er einer der folgenden Formeln entspricht:

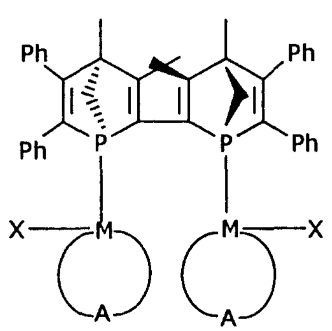
(VIIIa)

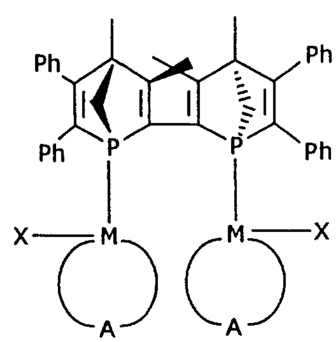
(VIIIb)

wobei in den genannten Formeln M Palladium oder Platin, X ein Halogenatom, vorzugsweise Chlor, und A den Rest eines chiralen metallischen Komplexes bedeutet, der einer der Formeln (VII) und vorzugsweise (VII') nach einem der Ansprüche 3 bis 6 entspricht.

**16.** Komplex mit einem optisch aktiven Diphosphin und einem Übergangsmetall, dadurch gekennzeichnet, daß der Ligand einer der folgenden Formeln entspricht:

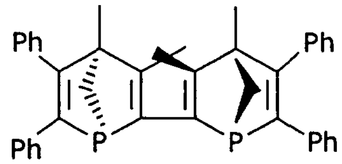

(Ia) - (S,S) - (+)

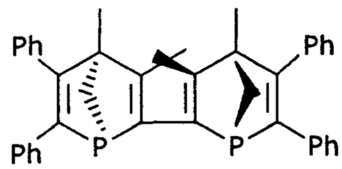

(Ib) - (R,R) - (-)

**17.** Komplex nach Anspruch 16, dadurch gekennzeichnet, daß das Übergangsmetall gewählt ist aus: Rhodium, Ruthenium, Rhenium, Iridium, Kobalt, Nickel, Platin, Palladium.

**18.** Komplex mit einem optisch aktiven Diphosphin und Rhodium und/oder Iridium nach Anspruch 16 oder 17, dadurch gekennzeichnet, daß er durch die folgenden Formeln dargestellt wird:

$$[M\ L_2(P^*P)]\ Y \qquad (IIa)$$

$$[M\ L_2(P^*P)]\ Y \qquad (IIb)$$

wobei in den genannten Formeln:

- (P*P) in der Formel (IIa) das Diphosphin der Formel (Ia) und in der Formel (IIb) das Diphosphin der Formel (Ib) bedeutet,
- M Rhodium oder Iridium bedeutet,
- Y einen koordinierenden Anionoliganden bedeutet,
- L einen neutralen Liganden bedeutet.

**19.** Komplex nach Anspruch 18, dadurch gekennzeichnet, daß er der Formel (IIa) oder (IIb) entspricht, in der:

- L ein Olefin mit 2 bis 12 Kohlenstoffatomen und zwei Liganden bedeutet, wobei L zwischen ihnen gebunden sein kann, um eine lineare oder cyclische polyungesättigte Kohlenwasserstoffkette zu bilden; L bedeutet vorzugsweise 1,5-Cyclooctadien, Norbornadien, Ethylen,
- Y ein $PF_6^-$-, $PCl_6^-$-, $BF_4^-$-, $BCl_4^-$-, $SbF_6^-$-, $SbCl_6^-$-, $BPh_4^-$-, $ClO_4^-$-, $CN^-$-, $CF_3SO_3^-$- oder Halogen-Anion, vorzugsweise ein $CL^-$- oder $Br^-$-Anion, ein 1,3-Dicetonat-, Alkylcarboxylat- oder Haloalkylcarboxylat-Anion mit einem niedrigen Alkylrest, ein Phenylcarboxylat- oder Phenolat-Anion bedeutet, dessen Benzolring mit niedrigen Alkylresten und/oder Halogenatomen substituiert sein kann.

**20.** Komplex mit einem optisch aktiven Diphosphin und Iridium nach Anspruch 16 oder 17, dadurch gekennzeichnet, daß er durch die folgenden Formeln dargestellt wird:

$$[Ir\ L\ (P^*P)]\ Y \qquad (IIIa)$$

$$[Ir\ L\ (P^*P)\ Y] \qquad (IIIb)$$

wobei in den genannten Formeln (P*P), L und Y die Bedeutungen wie in den Formeln (IIa) und (IIb) haben.

**21.** Komplex mit einem optisch aktiven Diphosphin und Ruthenium nach Anspruch 16 oder 17, dadurch gekennzeichnet, daß er durch die folgenden Formeln dargestellt wird:

$$[RuY_1Y_2(P^*P)] \quad (IVa)$$

$$[RuY_1Y_2(P^*P)] \quad (IVb)$$

wobei in den genannten Formeln:

- (P*P) in der Formel (IVa) das Diphosphin der Formel (Ia) und in der Formel (IVb) das Diphosphin der Formel (Ib) bedeutet,
- die gleichen oder verschiedenen $Y_1$ und $Y_2$ vorzugsweise ein $PF_6^-$-, $PCl_6^-$-, $BF_4^-$-, $BCl_4^-$-, $SbF_6^-$-, $SbCl_6^-$-, $BPh_4^-$-, $ClO_4^-$-, $CF_3SO_3^-$-Anion, ein Halogenatom, noch spezieller Chlor oder Brom, oder ein Carboxylat-Anion bedeuten, vorzugsweise ein Acetat- oder Trifluoressigsäureester-Anion.

**22.** Komplex mit einem optisch aktiven Diphosphin und Ruthenium nach Anspruch 16 oder 17, dadurch gekennzeichnet, daß er durch die folgenden Formeln dargestellt wird:

$$[RuY_1Ar(P^*P)Y_2] \quad (IVc)$$

$$[RuY_1Ar(P^*P)Y_2] \quad (IVd)$$

wobei in den genannten Formeln:

- ($P^*P$) in der Formel (IVc) das Diphosphin der Formel (Ia) und in der Formel (IVd) das Diphosphin der Formel (Ib) bedeutet,
- Ar Benzol, p-Methylisopropylbenzol, Hexamethylbenzol bedeutet,
- $Y_1$ ein Halogenatom bedeutet, vorzugsweise Chlor oder Brom,
- $Y_2$ ein Anion bedeutet, vorzugsweise ein $PF_6^-$-, $PCl_6^-$-, $BF_4^-$-, $BCl_4^-$-, $SbF_6^-$-, $SbCl_6^-$ -, $BPh_4^-$-, $ClO_4^-$-, $CF_3SO_3^-$-Anion.

**23.** Verfahren zur Herstellung eines Komplexes nach einem der Ansprüche 16 bis 22, dadurch gekennzeichnet, daß es darin besteht, ein Diphosphin der Formel (Ia) oder (Ib) mit einer Übergangsmetallverbindung in einem geeigneten organischen Lösungsmittel reagieren zu lassen.

**24.** Verwendung eines in einem der Ansprüche 16 bis 22 beschriebenen Komplexes in einer asymmetrischen Katalyse.

**25.** Verwendung eines in einem der Ansprüche 16 bis 22 beschriebenen Komplexes in einer asymmetrischen Hydrierung.

**26.** Verfahren zur Herstellung einer optisch aktiven Carboxylsäure und/oder eines optisch aktiven Derivats, dadurch gekennzeichnet, daß man die asymmetrische Hydrierung einer $\alpha,\beta$-ungesättigten Carboxylsäure und/oder ihrer Derivate in Gegenwart einer wirksamen Menge eines metallischen Komplexes durchführt, der als Liganden das optisch aktive Diphosphin der Formel (Ia) oder (Ib) und ein Übergangsmetall enthält.

**27.** Verfahren nach Anspruch 26, dadurch gekennzeichnet, daß die $\alpha,\beta$-ungesättigte Carboxylsäure und/oder das Derivat der folgenden allgemeinen Formel entspricht:

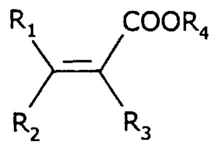

(V)

wobei in der genannten Formel (V):

- $R_1$, $R_2$, $R_3$ und $R_4$ ein Wasserstoffatom oder irgendeine Kohlenwasserstoffgruppe mit der Maßgabe bedeuten, daß:

  • wenn $R_1$ unterschiedlich zu $R_2$ ist, dann kann $R_3$ irgendeine mit ℛ bezeichnete Kohlenwasserstoffgruppe oder funktionelle Gruppe sein,
  • wenn $R_1$ oder $R_2$ ein Wasserstoffatom bedeuten und wenn $R_1$ unterschiedlich zu $R_2$ ist, dann ist $R_3$ unterschiedlich zu einem Wasserstoffatom und unterschiedlich zu -$COOR_4$,
  • wenn $R_1$ gleich mit $R_2$ ist und irgendeine mit ℛ bezeichnete Kohlenwasserstoffgruppe oder funktionelle Gruppe bedeutet, dann ist $R_3$ unterschiedlich zu -CH-( ℛ )$_2$ und unterschiedlich zu -$COOR_4$,

- eine der Gruppen $R_1$, $R_2$ und $R_3$ eine funktionelle Gruppe bedeuten können.

**28.** Verfahren nach Anspruch 27, dadurch gekennzeichnet, daß die α,β-ungesättigte Carboxylsäure und/oder das Derivat der Formel (V) entspricht, in der die gleichen oder verschiedenen Reste $R_1$ bis $R_4$ einen gegebenenfalls substituierten Kohlenwasserstoffrest mit 1 bis 20 Kohlenstoffatomen bedeuten, der ein aliphatischer acyclischer gesättigter oder ungesättigter, linearer oder verzweigter Rest sein kann; der ein carbocyclischer oder heterocyclischer gesättigter, ungesättigter oder aromatischer, monocyclischer oder polycyclischer Rest sein kann; der ein aliphatischer gesättigter oder ungesättigter, linearer oder verzweigter Rest sein kann, der Träger eines cyclischen Substituenten ist.

**29.** Verfahren nach Anspruch 26, dadurch gekennzeichnet, daß die verwendete Carboxylsäure eine substituierte Acrylsäure ist, die Ausgangsstoff einer Aminosäure ist.

**30.** Verfahren nach Anspruch 29, dadurch gekennzeichnet, daß die verwendete Carboxylsäure eine substituierte Acrylsäure ist, die Ausgangsstoff einer Aminosäure und/oder eines Derivats ist, die/das der folgenden Formel entspricht:

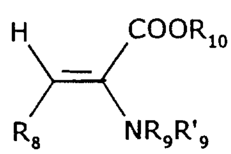

(Va)

wobei in der genannten Formel (Va):

- die gleichen oder verschiedenen $R_9$, $R'_9$ ein Wasserstoffatom, eine lineare oder verzweigte Alkylgruppe mit 1 bis 12 Kohlenstoffatomen, eine Phenylgruppe oder eine Acylgruppe mit 2 bis 12 Kohlenstoffatomen, vorzugsweise eine Acetyl- oder Benzoylgruppe, bedeuten,
- $R_8$ ein Wasserstoffatom, eine Alkylgruppe mit 1 bis 12 Kohlenstoffatomen, einen Cycloalkylrest mit 3 bis 8 Kohlenstoffatomen, einen Arylalkylrest mit 6 bis 12 Kohlenstoffatomen, einen Arylrest mit 6 bis 12 Kohlenstoffatomen, einen heterocyclischen Rest mit 4 bis 7 Kohlenstoffatomen bedeutet,
- $R_{10}$ ein Wasserstoffatom oder eine lineare oder verzweigte Alkylgruppe mit 1 bis 4 Kohlenstoffatomen bedeutet.

**31.** Verfahren nach Anspruch 29 oder 30, dadurch gekennzeichnet, daß die verwendete Carboxylsäure N-Acetyl α-Amino β-Phenylacryl-Säure, N-Benzoyl α-Amino β-Phenylacryl-Säure, in denen der Phenylring gegebenenfalls mit einer oder mehreren Alkyl-, Alkoxy- (?) oder Hydroxygruppen substituiert ist, N-Acetyl α-Amino β-Indolylacrylsäure, N-Benzoyl α-Amino β-Indolylacrylsäure, N-Acetyl α-Amino β-Isobutyl Acrylsäure ist.

**32.** Verfahren nach Anspruch 26, dadurch gekennzeichnet, daß die verwendete Carboxylsäure Itaconsäure und/oder ein Derivat ist, die/das der folgenden Formel entspricht:

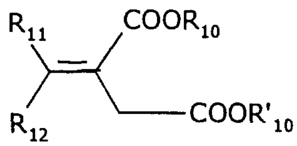

(Vb)

wobei in der genannten Formel (Vb):

- die gleichen oder verschiedenen $R_{11}$ oder $R_{12}$ ein Wasserstoffatom, eine lineare oder verzweigte Alkylgruppe mit 1 bis 12 Kohlenstoffatomen, einen Cycloalkylrest mit 3 bis 8 Kohlenstoffatomen, einen Arylalkylrest mit 6 bis 12 Kohlenstoffatomen, einen Arylrest mit 6 bis 12 Kohlenstoffatomen, einen heterocyclischen Rest mit 4 bis 7 Kohlenstoffatomen bedeuten,
- die identischen oder verschiedenen $R_{10}$, $R'_{10}$ ein Wasserstoffatom oder eine lineare oder verzweigte Alkylgruppe mit 1 bis 4 Kohlenstoffatomen bedeuten.

**33.** Verfahren nach Anspruch 32, dadurch gekennzeichnet, daß die verwendete Carboxylsäure Itaconsäure oder Dimethylitaconat ist.

**34.** Verfahren nach Anspruch 26, dadurch gekennzeichnet, daß die verwendete Carboxylsäure ein Substrat ist, das der folgenden Formel entspricht:

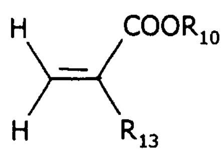

(Vc)

wobei in der genannten Formel (Vc):

- $R_{10}$ ein Wasserstoffatom oder eine lineare oder verzweigte Alkylgruppe mit 1 bis 4 Kohlenstoffatomen bedeutet,
- $R_{13}$ eine Phenyl- oder Naphthylgruppe bedeutet, die gegebenenfalls Träger eines Substituenten oder mehrerer Substituenten R ist:
  - R kann $R_0$, eine der folgenden Gruppen, bedeuten:
    - eine lineare oder verzweigte Alkyl- oder Alkenylgruppe mit 1 bis 12 Kohlenstoffatomen, vorzugsweise eine lineare oder verzweigte Alkylgruppe mit 1 bis 4 Kohlenstoffatomen,
    - eine lineare oder verzweigte Alkoxygruppe mit 1 bis 12 Kohlenstoffatomen, vorzugsweise eine lineare oder verzweigte Alkoxygruppe mit 1 bis 4 Kohlenstoffatomen,
    - eine lineare oder verzweigte Acyloxygruppe mit 2 bis 8 Kohlenstoffatomen, vorzugsweise eine Acetoxygruppe,
    - eine lineare oder verzweigte Acylamidogruppe mit 1 bis 8 Kohlenstoffatomen, vorzugsweise eine Acetamidogruppe,
    - eine $NO_2$-Gruppe,
  - R kann $R_0'$, eine der folgenden komplexeren Gruppen, bedeuten:
    - eine Gruppe der Formel

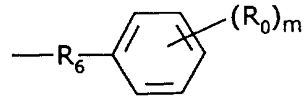

in der:

- $R_6$ ein valentes Bindemittel bedeutet; eine divalente, lineare oder verzweigte, gesättigte oder ungesättigte Kohlenwasserstoffgruppe mit 1 bis 6 Kohlenstoffatomen, wie z.B. Methylen, Ethylen, Propylen, Isopropylen, Isopropyliden oder eine der folgenden mit Z bezeichneten Gruppen: -O-; -CO-; -COO-, $-NR_7-$; $-CO-NR_7-$; -S-; $-SO_2-$; $-NR_7-CO-$; wobei in den genannten Formeln $R_7$ ein Wasserstoffatom, eine lineare oder verzweigte Alkylgruppe mit 1 bis 6 Kohlenstoffatomen bedeutet,

- $R_0$ hat die zuvor gegebene Bedeutung,
- m ist eine ganze Zahl von 0 bis 4.

**35.** Verfahren nach Anspruch 34, dadurch gekennzeichnet, daß die verwendete Carboxylsäure 2-(3-Benzoylphenyl) propionsäure, 2-(4-Isobutylphenyl)propionsäure, 2-(5-Methoxynapthyl)propionsäure ist.

**36.** Verfahren nach einem der Ansprüche 26 bis 35, dadurch gekennzeichnet, daß die Hydrierung bei einer Temperatur zwischen 20 und 100 °C durchgeführt wird.

**37.** Verfahren nach einem der Ansprüche 26 bis 36, dadurch gekennzeichnet, daß der Wasserstoffdruck zwischen 0,1 und 200 bar und noch spezieller zwischen 1 und 150 bar liegen kann.

**38.** Verfahren nach einem der Ansprüche 26 bis 37, dadurch gekennzeichnet, daß man mindestens einen Komplex nach einem der Ansprüche 16 bis 22 verwendet.

**39.** Verfahren nach einem der Ansprüche 26 bis 38, dadurch gekennzeichnet, daß das Verhältnis zwischen der Anzahl der in dem Komplex enthaltenen Metallatome und der Anzahl der Mole der zu hydrierenden Verbindung zwischen 0,1 und 0,0001 liegt.

**40.** Verfahren nach einem der Ansprüche 26 bis 39, dadurch gekennzeichnet, daß man nach Bildung des hydrierten Komplexes eine basische Verbindung hinzugibt.

## Claims

**1.** Optically active bis[1-phospha-2,3-diphenyl-4,5-dimethylnorbornadiene] diphosphines corresponding to the following formulae:

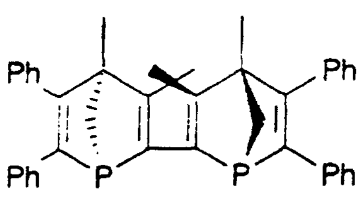

(S,S) - (+) - (Ia)

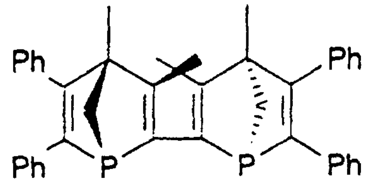

(R,R) - (-) - (Ib)

2. Process for resolving the racemic bis[1-phospha-2,3-diphenyl-4,5-dimethylnorbornadiene] mixture which comprises reacting it with a palladium and/or platinum complex as chiral auxiliary, in an organic solvent, thus forming diastereoisomer complexes, and in then resolving the said optically pure complexes.

3. Process according to Claim 2, characterized in that the chiral auxiliary corresponds to the general formula (VII) :

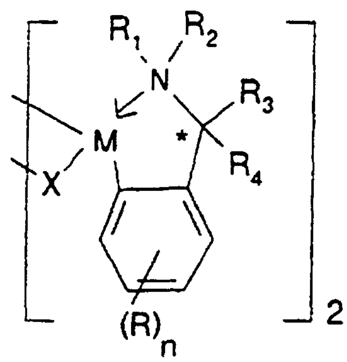

(VII)

in the said formula:

- M represents palladium and/or platinum,
- $R_1$, $R_2$, $R_3$ and $R_4$ represent a hydrogen atom or an alkyl radical having from 1 to 10 carbon atoms or a cycloalkyl radical having from 3 to 10 carbon atoms,
- $R_3$ and $R_4$ are different and at least one of the two represents a hydrogen atom,
- R has the meaning given for $R_1$, $R_2$, $R_3$ and $R_4$,
- X represents a halogen atom,
- n is a number from 0 to 4,
- when n is greater than 1, two R radicals and the 2 successive atoms of the benzene ring can form, between them, a ring having from 5 to 7 carbon atoms.

4. Process according to either of Claims 2 and 3, characterized in that the chiral auxiliary corresponds to the general formula (VII) in which $R_1$, $R_2$, $R_3$ and $R_4$ represent a hydrogen atom or a methyl radical, X represents a chlorine atom and n is equal to 0.

5. Process according to either of Claims 2 and 3, characterized in that the chiral auxiliary corresponds to the general formula (VII) in which $R_1$, $R_2$, $R_3$ and $R_4$ represent a hydrogen atom or a methyl radical, X represents a chlorine atom and, when n is equal to 2, two R radicals form a benzene ring.

6. Process according to one of Claims 2 to 4, characterized in that the chiral auxiliary corresponds to the general formula (VII'):

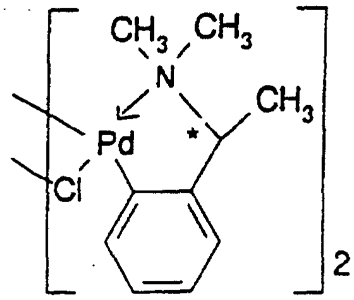

(VII')

7. Process according to one of Claims 2 to 6, characterized in that the amount of complex of the abovementioned metals, expressed as metal, is from 0.5 to 1 metal atom per phosphorus atom.

8. Process according to one of Claims 2 to 7, characterized in that the organic solvent is chosen from:

- aliphatic hydrocarbons and more particularly paraffins such as, in particular, pentane, hexane, heptane, octane, isooctane, nonane, decane, undecane, tetradecane, petroleum ether and cyclohexane; aromatic hydrocarbons such as, in particular, benzene, toluene, xylenes, ethylbenzene, diethylbenzenes, trimethylbenzenes, cumene, pseudocumene or petroleum fractions composed of a mixture of alkylbenzenes, in particular fractions of Solvesso® type,
- halogenated aliphatic or aromatic hydrocarbons, and mention may be made of: perchlorinated hydrocarbons such as, in particular, trichloromethane or tetrachloroethylene; partially chlorinated hydrocarbons, such as dichloromethane, dichloroethane, tetrachloroethane, trichloroethylene, 1-chlorobutane or 1,2-dichlorobutane; or monochlorobenzene, 1,2-dichlorobenzene, 1,3-dichlorobenzene, 1,4-dichlorobenzene or mixtures of different chlorobenzenes.

9. Process according to Claim 8, characterized in that the organic solvent is benzene or toluene.

10. Process according to one of Claims 2 to 9, characterized in that the concentration of the diphosphine in the reaction solvent is preferably between 0.05 and 1 mol/litre and more particularly still between 0.05 and 0.2 mol/litre.

11. Process according to one of Claims 2 to 10, characterized in that the separation is carried out at ambient temperature, generally under a controlled atmosphere of inert gases, preferably under nitrogen.

12. Process according to one of Claims 2 to 11, characterized in that the two enantiomers are separated by liquid chromatography on a column with, preferably, a support made of silica.

13. Process according to Claim 12, characterized in that the column is eluted with a mixture of suitable solvents, preferably a toluene/ethyl acetate mixture preferentially comprising 80% by volume of toluene and 20% by volume of ethyl acetate.

14. Process according to Claim 13, characterized in that the two pure enantiomers of the diphosphine are recovered by dissolving the complexes in an organic solvent, such as, for example, dichloromethane, and by then decomplexing using a hydrocyanic acid salt, preferably an alkali metal salt and more preferentially still the sodium salt.

15. Complex comprising an optically active diphosphine and a chiral auxiliary, characterized in that it corresponds to one of the following formulae:

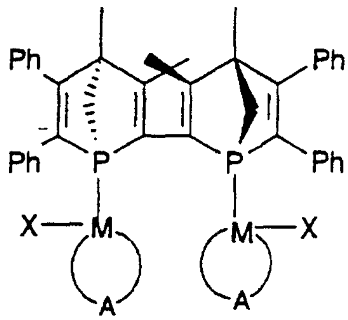

(VIII a)

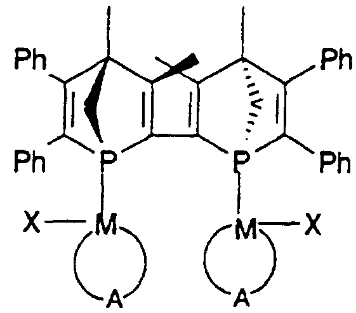

(VIII b)

in the said formulae M represents palladium or platinum, X a halogen atom, preferably chlorine, and A symbolizes the residue of a chiral metal complex corresponding to one of the formulae (VII) and preferentially (VII') according to one of Claims 3 to 6.

**16.** Complex comprising an optically active diphosphine and a transition metal, characterized in that the ligand corresponds to one of the following formulae:

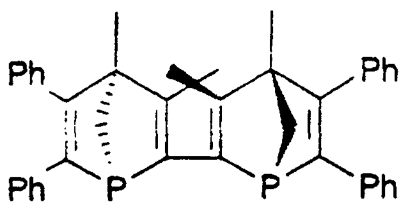

(S,S) - (+) - (Ia)

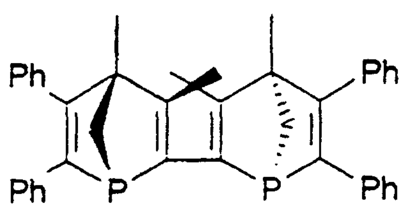

(R,R) - (-) - (Ib)

**17.** Complex according to Claim 16, characterized in that the transition metal is chosen from: rhodium, ruthenium, rhenium, iridium, cobalt, nickel, platinum or palladium.

**18.** Complex comprising an optically active diphosphine and rhodium and/or iridium according to one of Claims 16 and 17, characterized in that it is represented by the following formulae:

$$[M\ L_2(P^*P)]\ Y \qquad (IIa)$$

$$[M\ L_2(P^*P)]Y \qquad (IIb)$$

in the said formulae:

- (P*P) represents, in the formula (IIa), the diphosphine of formula (Ia) and, in the formula (IIb), the diphosphine of formula (Ib),

- M represents rhodium or iridium,
- Y represents an anionic coordinating ligand,
- L represents a neutral ligand.

**19.** Complex according to Claim 18, characterized in that it corresponds to the formula (IIa) or (IIb) in which:

- L represents an olefin having from 2 to 12 carbon atoms and two L ligands can be joined to one another in order to form a linear or cyclic polyunsaturated hydrocarbonaceous chain; L preferably representing 1,5-cyclooctadiene, norbornadiene or ethylene,
- Y represents a $PF_6^-$, $PCl_6^-$, $BF_4^-$, $BCl_4^-$, $SbF_6^-$, $SbCl_6^-$, $BPh_4^-$, $ClO_4^-$, $CN^-$ or $CF_3SO_3^-$ anion, halogen, preferably $Cl^-$ or $Br^-$, a 1,3-diketonate, alkylcarboxylate or haloalkylcarboxylate anion with a lower alkyl radical, or a phenylcarboxylate or phenoxide anion in which the benzene ring can be substituted by lower alkyl radicals and/ or halogen atoms.

**20.** Complex comprising an optically active diphosphine and iridium according to either of Claims 16 and 17, characterized in that it is represented by the following formulae:

$$[IrL(P^*P)]Y \quad \text{(IIIa)}$$

$$[IrL(P^*P)]Y \quad \text{(IIIb)}$$

in the said formulae (P*P), L and Y have the meanings given for the formulae (IIa) and (IIb).

**21.** Complex comprising an optically active diphosphine and ruthenium according to either of Claims 16 and 17, characterized in that it is represented by the following formulae:

$$[RuY_1Y_2(P^*P)] \quad \text{(IVa)}$$

$$[RuY_1Y_2(P^*P)] \quad \text{(IVb)}$$

in the said formulae:

- (P*P) represents, in the formula (IVa), the diphosphine of formula (Ia) and, in the formula (IVb), the diphosphine of formula (Ib),
- $Y_1$ and $Y_2$, which are identical or different, preferably represent a $PF_6^-$, $PCl_6^-$, $BF_4^-$, $BCl_4^-$, $SbF_6^-$, $SbCl_6^-$, $BPh_4^-$, $ClO_4^-$ or $CF_3SO_3^-$ anion, a halogen atom, more particularly chlorine or bromine, or a carboxylate anion, preferentially acetate or trifluoroacetate.

**22.** Complex comprising an optically active diphosphine and ruthenium according to one of Claims 16 and 17, characterized in that it is represented by the following formulae:

$$[RuY_1Ar(P^*P)Y_2] \quad \text{(IVc)}$$

$$[RuY_1Ar(P^*P)Y_2] \quad \text{(IVd)}$$

in the said formulae:

- (P*P) represents, in the formula (IVc), the diphosphine of formula (Ia) and, in the formula (IVd), the diphosphine of formula (Ib),
- Ar represents benzene, p-methylisopropylbenzene or hexamethylbenzene,
- $Y_1$ represents a halogen atom, preferably chlorine or bromine,

- $Y_2$ represents an anion, preferably $PF_6^-$, $PCl_6^-$, $BF_4^-$, $BCl_4^-$, $SbF_6^-$, $SbCl_6^-$, $BPh_4^-$, $ClO_4^-$ or $CF_3SO_3^-$.

**23.** Process for the preparation of a complex according to one of Claims 16 to 22, characterized in that it comprises reacting a diphosphine of formula (Ia) or (Ib) with the transition metal compound in a suitable organic solvent.

**24.** Use of a complex described in one of Claims 16 to 22 in asymmetric catalysis.

**25.** Use of a complex described in one of Claims 16 to 22 in asymmetric hydrogenation.

**26.** Process for the preparation of an optically active carboxylic acid and/or derivative, characterized in that an α,β-unsaturated carboxylic acid and/or its derivatives is/are asymmetrically hydrogenated in the presence of an effective amount of a metal complex comprising, as ligand, the optically active diphosphine of formula (Ia) or (Ib) and a transition metal.

**27.** Process according to Claim 26, characterized in that the α,β-unsaturated carboxylic acid and/or derivative corresponds to the following general formula:

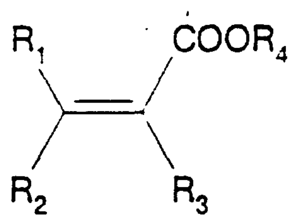

(V)

in the said formula (V):

- $R_1$, $R_2$, $R_3$ and $R_4$ represent a hydrogen atom or any hydrocarbonaceous group, insofar as:
    - if $R_1$ is other than $R_2$ and other than a hydrogen atom, then $R_3$ can be any functional or hydrocarbonaceous group denoted by R,
    - if $R_1$ or $R_2$ represents a hydrogen atom and if $R_1$ is other than $R_2$, then $R_3$ is other than a hydrogen atom and other than $-COOR_4$,
    - if $R_1$ is identical to $R_2$ and represents any functional or hydrocarbonaceous group denoted by *R*, then $R_3$ is other than $-CH-(R)_2$ and other than $-COOR_4$,

- it being possible for one of the $R_1$, $R_2$ and $R_3$ groups to represent a functional group.

**28.** Process according to Claim 27, characterized in that the α,β-unsaturated carboxylic acid and/or derivative corresponds to the formula (V) in which the $R_1$ to $R_4$ radicals, which are identical or different, represent an optionally substituted hydrocarbonaceous radical having from 1 to 20 carbon atoms which can be a linear or branched, saturated or unsaturated, acyclic aliphatic radical; a monocyclic or polycyclic, saturated, unsaturated or aromatic, heterocyclic or carbocyclic radical; or a linear or branched, saturated or unsaturated, aliphatic radical carrying a cyclic substituent.

**29.** Process according to Claim 26, characterized in that the carboxylic acid used is a substituted acrylic acid which is a precursor of an amino acid.

**30.** Process according to Claim 29, characterized in that the carboxylic acid used is a substituted acrylic acid which is a precursor of an amino acid and/or derivative corresponding to the following formula:

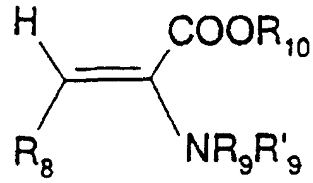

(Va)

in the said formula (Va):

- $R_9$ and $R'_9$, which are identical or different, represent a hydrogen atom, a linear or branched alkyl group having from 1 to 12 carbon atoms, a phenyl group or an acyl group having from 2 to 12 carbon atoms, preferably an acetyl or benzoyl group,
- $R_8$ represents a hydrogen atom, an alkyl group having from 1 to 12 carbon atoms, a cycloalkyl radical having from 3 to 8 carbon atoms, an arylalkyl radical having from 6 to 12 carbon atoms, an aryl radical having from 6 to 12 carbon atoms or a heterocyclic radical having from 4 to 7 carbon atoms,
- $R_{10}$ represents a hydrogen atom or a linear or branched alkyl group having from 1 to 4 carbon atoms.

**31.** Process according to Claim 29 or 30, characterized in that the carboxylic acid used is N-acetyl-α-amino-β-phenylacrylic acid, N-benzoyl-α-amino-β-phenylacrylic acid, in which the phenyl ring is optionally substituted by one or a number of alkyl, alkyloxy or hydroxyl groups, N-acetyl-α-amino-β-indolylacrylic acid, N-benzoyl-α-amino-β-indolylacrylic acid or N-acetyl-α-amino-β-isobutylacrylic acid.

**32.** Process according to Claim 26, characterized in that the carboxylic acid used is itaconic acid and/or derivative corresponding to the following formula:

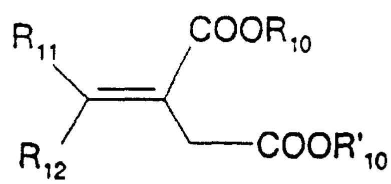

(Vb)

in the said formula (Vb):

- $R_{11}$ and $R_{12}$, which are identical or different, represent a hydrogen atom, a linear or branched alkyl group having from 1 to 12 carbon atoms, a cycloalkyl radical having from 3 to 8 carbon atoms, an arylalkyl radical having from 6 to 12 carbon atoms, an aryl radical having from 6 to 12 carbon atoms or a heterocyclic radical having from 4 to 7 carbon atoms,
- $R_{10}$ and $R'_{10}$, which are identical or different, represent a hydrogen atom or a linear or branched alkyl group having from 1 to 4 carbon atoms.

**33.** Process according to Claim 32, characterized in that the carboxylic acid used is itaconic acid or dimethyl itaconate.

**34.** Process according to Claim 26, characterized in that the carboxylic acid used is a substrate corresponding to the following formula:

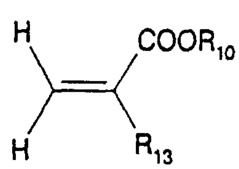

(Vc)

in the said formula (Vc):

- $R_{10}$ represents a hydrogen atom or a linear or branched alkyl group having from 1 to 4 carbon atoms,
- $R_{13}$ represents a phenyl or naphthyl group, optionally carrying a substituent or a number of substituents R:

  - R can represent $R_0$, one of the following groups:

    - a linear or branched alkyl or alkenyl group having from 1 to 12 carbon atoms, preferably a linear or branched alkyl group having from 1 to 4 carbon atoms,
    - a linear or branched alkoxy group having from 1 to 12 carbon atoms, preferably a linear or branched alkoxy group having from 1 to 4 carbon atoms,
    - a linear or branched acyloxy group having from 2 to 8 carbon atoms, preferably an acetoxy group,
    - a linear or branched acylamido group having from 1 to 8 carbon atoms, preferably an acetamido group,
    - an $NO_2$ group,

- R can represent $R_0'$, one of the following, more complex groups:

  - a group of formula

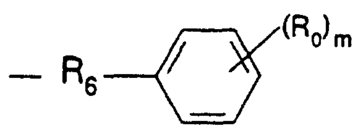

  in which:

    - $R_6$ represents a valence bond; a saturated or unsaturated, linear or branched, divalent hydrocarbonaceous group having from 1 to 6 carbon atoms such as, for example, methylene, ethylene, propylene, isopropylene or isopropylidene or one of the following groups known as Z:
      -O-; -CO-; -COO-; $-NR_7-$; $-CO-NR_7-$; -S-; $-SO_2-$ or $-NR_7-CO-$;
      in the said formulae $R_7$ represents a hydrogen atom or a linear or branched alkyl group having from 1 to 6 carbon atoms,
    - $R_0$ has the meaning given above,
    - m is an integer from 0 to 4.

**35.** Process according to Claim 34, characterized in that the carboxylic acid used is 2-(3-benzoylphenyl) propionic acid, 2-(4-isobutylphenyl)propionic acid or 2-(5-methoxynaphthyl)propionic acid.

**36.** Process according to one of Claims 26 to 35, characterized in that the hydrogenation is generally carried out at a temperature of between 20 and 100°C.

**37.** Process according to one of Claims 26 to 36, characterized in that the hydrogen pressure can be between 0.1 and 200 bar and more preferentially between 1 and 150 bar.

**38.** Process according to one of Claims 26 to 37, characterized in that use is made of at least one complex according to one of Claims 16 to 22.

**39.** Process according to one of Claims 26 to 38, characterized in that the ratio of the number of atoms of metal present in the complex to the number of moles of the compound to be hydrogenated is between 0.1 and 0.0001.

**40.** Process according to one of Claims 26 to 39, characterized in that, after the formation of the hydrogenation complex, a basic compound is added.